# EUROPÄISCHE PATENTSCHRIFT

(11) **EP 1 926 525 B1**
(45) Veröffentlichungstag und Bekanntmachung des Hinweises auf die Patenterteilung: **26.05.2010**
(21) Anmeldenummer: 06777168.3
(22) Anmeldetag: 05.09.2006
(51) Int. Cl.: A61N 1/36

(54) **ERZEUGEN EINES KOMBINATIONSSIGNALS FÜR COCHLEA-IMPLANTATE**
GENERATION OF A COMPOSITE SIGNAL FOR COCHLEAR IMPLANTS
GÉNÉRATION D'UN SIGNAL COMPOSITE POUR IMPLANTS COCHLÉAIRES

(30) Priorität: 19.09.2005 DE 102005044628; 13.10.2005 DE 102005049507
(43) Veröffentlichungstag der Anmeldung: 04.06.2008
(73) Patentinhaber: Fraunhofer-Gesellschaft zur Förderung der angewandten Forschung e.V., 80686 München (DE)
(72) Erfinder: EDLER, Bernd, 30419 Hannover (DE); BÜCHNER, Andreas, 30916 Isenhagen (DE); NOGUEIRA, Waldo, 1020 Laken, Brüssel (BE); KLEFENZ, Frank, 68159 Mannheim (DE)
(74) Vertreter: Zinkler, Franz
(86) Internationale Anmeldenummer: PCT/EP2006/008650
(87) Internationale Veröffentlichungsnummer: WO 2007/033762

(56) Entgegenhaltungen:
- WO-A-01/99470
- WO-A-96/12383
- WO-A-98/16086
- US-B1- 6 249 704
- US-B1- 6 728 578

## Beschreibung

Die vorliegende Erfindung bezieht sich im Allgemeinen auf ein Cochlea-Implantat, auf eine Vorrichtung zum Erzeugen eines Steuersignals für ein Cochlea-Implantat, auf eine Vorrichtung zum Erzeugen eines Kombinationssignals, auf ein Kombinationssignal sowie auf entsprechende Verfahren, im Speziellen auf ein Konzept zur Übertragung und Speicherung von Audiosignalen für Cochlea-Implantate.

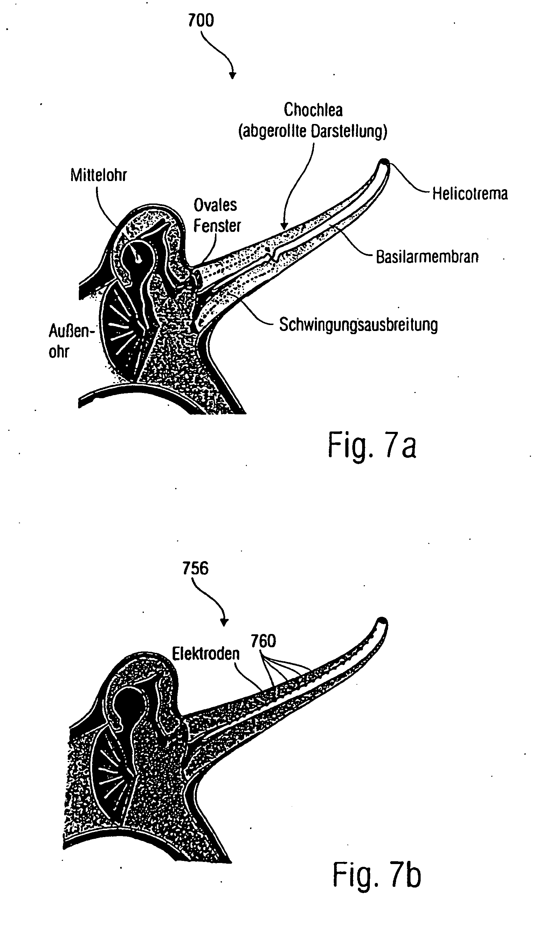

Im Folgenden werden zunächst die Grundlagen hinsichtlich des Betriebs von Cochlea-Implantaten erläutert, um ein Verständnis der vorliegenden Erfindung zu verbessern bzw. zu erleichtern. Patienten mit vollständigem Verlust des Gehörsinns erhalten häufig sogenannte Cochlea-Implantate, die eine Erzeugung von Schallreizen durch elektrische Impulse im Innenohr ermöglichen. Zum besseren Verständnis zeigt die Fig. 7a eine schematische Darstellung eines menschlichen Ohres, wobei die Cochlea in einer abgerollten Form dargestellt ist. Wie es aus der schematischen Darstellung 700 der Fig. 7a ersichtlich ist, erzeugt ein Schallreiz eine Schwingung auf einer Basilarmembran der Cochlea die mit Hilfe von Härchen und zugehörigen Hörzellen in Nervenimpulse umgewandelt wird. In einem gesunden Gehör erfolgt in der Cochlea eine Umsetzung von Tonfrequenzen in Positionen entlang der Basilarmembran. In anderen Worten, abhängig von einer Frequenz eines eintreffenden Schallereignisses wird ein der Frequenz entsprechender Ortsbereich der Basilarmembran besonders stark angeregt.

Fig. 7b zeigt eine schematische Darstellung eines menschlichen Ohres, in das Elektroden eines Cochlea-Implantats eingebracht sind. Aus der graphischen Darstellung 750 der Fig. 7b ist ersichtlich, dass ein Cochlea-Implantat mehrere Elektroden 760 besitzt, die an verschiedenen Positionen entlang der Basilarmembran in der Cochlea platziert sind. Durch geeignete Impulsmuster, die an die Elektroden 760 angelegt werden, kann daher ein Cochlea-Implantat eine, wenn auch nur grobe, Annäherung der in einem gesunden Gehör auftretenden Reize vornehmen. Es sei hierbei darauf hingewiesen, dass ein Cochlea-Implantat typischerweise direkt einen Hörnerv bzw. mehrere Hörnerven anregt.

Ein herkömmliches Cochlea-Implantat weist dabei ein Mikrophon zur Schallaufnahme und eine Signalverarbeitungseinheit zur Umwandlung des Schallsignals in geeignete Stimulationsimpulse auf. Die Qualität der von einem Patienten, der ein Cochlea-Implantat trägt, wahrgenommenen Klänge ist dabei durch verschiedene Faktoren begrenzt. Zum Einen ist die Anzahl der implantierbaren Elektroden begrenzt, so dass eine Frequenzauflösung eines gesunden Ohrs nicht erreicht werden kann. Weiterhin ist eine Anzahl von pro Zeiteinheit erzeugbaren Impulsen durch einen Übertragungsweg zu den Elektroden stark begrenzt. Schließlich ist ferner die Leistungsfähigkeit der Signalverarbeitungseinheit üblicherweise durch die Begrenzung der Stromaufnahme zur Erzielung einer ausreichenden Betriebszeit bei vorgegebener Batteriekapazität begrenzt.

Im Folgenden wird eine herkömmliche Signalverarbeitung für Cochlea-Implantat beschrieben. Derzeit übliche Cochlea-Implantate besitzen bis zu 22 Elektroden, die jeweils durch elektrische Impulse aktiviert werden können (Stimulation). Die jeweilige Impulsstärke eines elektrischen Impulses bestimmt die Stärke des Reizes, der über den Hörnerv zum Gehirn weitergeleitet wird. Die Position einer Elektrode entspricht hierbei der Tonhöhe bzw. Frequenz eines Tones, der in einem gesunden Gehör an der entsprechenden Stelle, also an der Position der Elektrode, eine maximale Auslegung der Basilarmembran der Cochlea hervorrufen würde. Daher wird herkömmlicherweise in der Signalverarbeitungseinheit das ankommende Tonsignal durch Bandpass-Filter in die entsprechenden Frequenzanteile zerlegt, wofür beispielsweise eine Filterbank verwendet wird. Aus einer Signalstärke in den zugehörigen Frequenzbändern kann dann die erforderliche Impulsstärke für Elektroden an verschiedene Positionen abgeleitet werden.

Da herkömmlicherweise zu jedem Zeitpunkt nur eine Elektrode stimuliert werden kann, erfolgt die Ansteuerung sequenziell, beispielsweise im Multiplex. Dabei kann die Reihenfolge der angesteuerten Elektroden variiert werden. Würden jeweils alle Elektroden nacheinander angesteuert, so ergäbe sich wegen der Begrenzung der Gesamt-Stimulationsrate für jede einzelne Elektrode eine zu geringe Zeitauflösung. Daher wurde ein Verfahren entwickelt, das in einem bestimmten Zeitabschnitt jeweils eine geringere Anzahl von Elektroden nach der entsprechenden Signalstärke auswählt. Begrenzt man bei einer Gesamtanzahl von M Elektroden die Stimulation in einem Zyklus auf N ausgewählte Elektroden, so spricht man von einem NofM-Strategie, die auch als fortgeschrittener kombinatorischer Codierer (ACE = Advanced Combinational Encoder) bezeichnet wird. Eine detaillierte Beschreibung der fortgeschrittenen kombinatorischen Codierung findet sich beispielsweise in dem Technischen Referenzhandbuch "ACE Speech Coding Strategy" (Nucleus Technical Reference Manual, Z43470 Issue3, Cochlear Corporation, Dezember 2002). Der Gesamt-Stimulationszyklus verkürzt sich. somit von M-Impulsen auf N-Impulse, was zu einer Erhöhung der Zeitauflösung führte. Übliche Zahlenwerte sind hierbei M = 22 und N = 8.

Die NofM-Strategie weist das Problem auf, dass die Frequenzbereiche mit den größten Signalstärken nicht immer die für die Wahrnehmung Wichtigsten sind. Daher wurden beispielsweise vom Laboratorium für Informationstechnologie der Universität Hannover (Deutschland) in Zusammenhang mit dem Hörzentrum Hannover (Deutschland) Verbesserungsansätze entwickelt, die zur Auswahl der Elektroden psychoakustische Modelle einsetzen, wie sie auch in der Audiocodierung zum Einsatz kommen. Für dieses neue Verfahren, dass auch als psychoakustischer fortgeschrittener kombinatorischer Codierer (PACE = Psychoacoustic Advanced Combinational Encoder) bezeichnet wird, reicht die Rechenleistung der bestehenden Signalverarbeitungseinheiten noch aus. Untersuchungen mit Patienten haben ferner angedeutet, dass der genannte Ansatz eine Sprachverständlichkeit erhöhen kann.

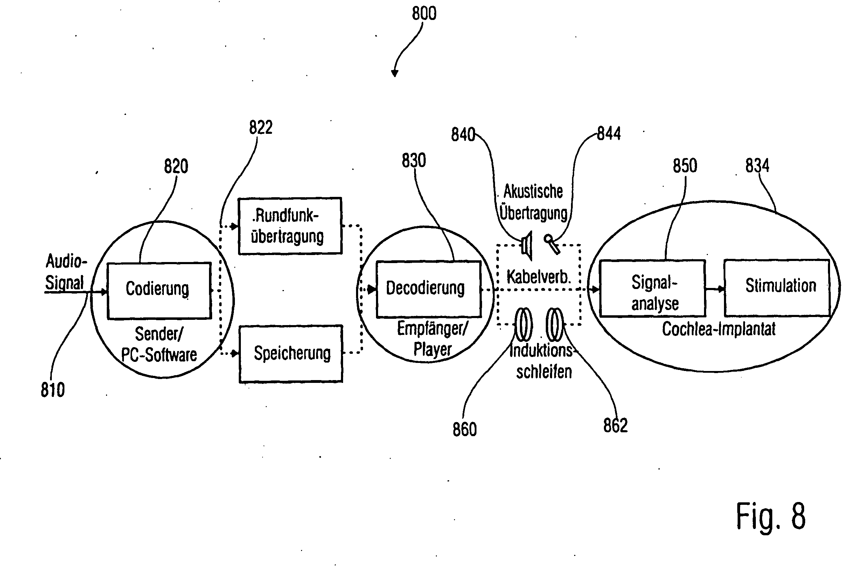

Fig. 8 zeigt eine schematische Darstellung von herkömmlichen Vorrichtungen zur Kopplung von Audiosignalen in ein Cochlea-Implantat. Die schematische Darstellung der Fig. 8 ist in ihrer Gesamtheit mit 800 bezeichnet. Die schematische Darstellung 800 beschreibt einen Übertragungsweg von Audiosignalen von einem Audiosignal-Ursprung bis in ein Cochlea-Implantat. Bei der heute üblichen digitalen Übertragung von Audiosignalen ist es üblich, ein Audiosignal 810 in einem Codierer 820 zu codieren. Der Codierer kann beispielsweise Teil eines (Rundfunk-) Senders sein und in Hardware oder Software realisiert sein. Ein codiertes Audiosignal 822 wird bei einer Rundfunkübertragung über eine Funkstrecke übertragen und dann von einem Empfänger 830 aufgenommen. In dem Empfänger erfolgt typischerweise eine Frequenzumsetzung und ferner eine Decodierung. Bei dem Empfänger kann es sich beispielsweise um einen Rundfunkempfänger oder einen Fernsehempfänger handeln. Für einen Radio- oder Fernsehempfang ist eine akustische Übertragung von dem Rundfunk- (Radio- oder Fernseh-) Empfänger 830 zu einer Signalverarbeitungseinheit 850 des Cochlea-Implantats 834 eine einfache und vorteilhafte Möglichkeit. In diesem Fall wird das Audiosignal durch den Rundfunkempfänger 830 über einen Lautsprecher 840 wiedergegeben. Das Cochlea-Implantat umfasst in dem genannten Fall ein Mikrophon 844, das das decodierte und durch den Lautsprecher 840 wiedergegebene Audiosignal aufnimmt und an die in dem Cochlea-Implantat befindliche Signalverarbeitungseinheit 850 weiterleitet. Die Signalverarbeitungseinheit 850 erzeugt daraufhin Stimulationsimpulse für die Anregung von Hörnerven aufgrund des von dem Mikrophon 844 gelieferten Audiosignals. Die Stimulationsimpulse können dann Elektroden zugeführt werden, die die Hörnerven eines menschlichen Patienten anregen.

Ferner wird es manchmal bevorzugt, ein decodiertes Audiosignal, das durch die Decodierung geliefert wird, direkt zu dem Cochlea-Implantat 834 zu übertragen. Für eine solche direkte Übertragung können beispielsweise Induktionsschleifen 860, 862 eingesetzt werden, wodurch die Übertragung unempfindlich gegenüber Störgeräuschen ist. Wird statt eines Rundfunkempfängers ein mobiles Abspielgerät wie beispielsweise ein portabler CD-Abspieler oder ein MP3-Abspieler verwendet, so kann herkömmlicher Weise das Audiosignal bzw. Tonsignal der Signalverarbeitungseinheit 850 des Cochlea-Implantats 834 auch direkt über ein Kabel zugeführt werden.

Zusammenfassend lässt sich also festhalten, dass das Cochlea-Implantat 834 herkömmlicher Weise ein Audiosignal empfängt, das durch einen Rundfunkempfänger oder ein Medien-Wiedergabegerät decodiert worden ist. Es hat sich allerdings gezeigt, dass das herkömmliche Konzept keine optimale Sprachqualität gewährleistet.

Die WO 01/99470 A1 beschreibt einen Audio-Prozessor für ein Cochlea-Implantat. Der Audioprozessor verwendet ein Modell einer Basilarmembranbewegung, um Stimuli basierend auf einer vorhergesagten Bewegung, die das akustische Signal in einer akustisch angeregten normal-hörenden Cochlea erzeugen würde, auszuwählen. Die verwendeten Filter decken mehrere Kanäle ab und überlappen. Somit erzeugen die Stimuli ein neurales Anregungsmuster, das eine räumlich-zeitlich wandernde Welle annähert, die an der Basilarmembran einer akustisch angeregten normal-hörenden Cochlea beobachtet wird.

Die US 6,728,578 B1 beschreibt eine Hüllkurven-basierte Amplituden-Abbildung für Cochlea-Implantat-Anregung. Eine Hüllkurven-basierte Amplitudenabbildung erreicht eine Signal-Kompression, die erforderlich ist, um einen natürlichen Schall-Pegel zu erreichen. Die Ausgangssignale einer Familie von parallelen Bandpassfiltern werden durch einen Hüllkurven-Detektor verarbeitet. Daraufhin erfolgt eine Dezimierung. Die resultierende Hüllkurve mit reduzierter Datenrate wird logarithmisch abgebildet, um einen Skalierungsfaktor für die ursprüngliche Bandpass gefilterte Ausgangssequenz mit hoher Datenrate zu erzeugen. Das resultierende skalierte Signal bestimmt den gegenwärtigen Pegel für die Stimulation der Cochlea für jedes Frequenzband.

Die US 6, 299, 704 B1 beschreibt eine Anregung, die eine Effizienz eines Cochlea-Implantats verbessert. Ein Cochlea-Stimulations-System umfasst eine Sprachverarbeitungsstrategie, die nicht-auditorisch-informative Stimuli sowie auditorisch-informative an die gleichen oder benachbarten Sätze von Elektroden anlegt. Die nicht-auditorisch informativen Stimuli werden an das auditorische Nervensystem angelegt, um die Eigenschaften und Antwortcharakteristika des auditorischen Systems zu beeinflussen. Die informationstragenden Stimuli werden an das auditorische Nervensystem zu der gleichen Zeit angelegt wie die nicht-auditorisch informativen Stimuli, oder werden während Zeitschlitzen zwischen der Anwendung der nicht-auditorisch-informativen Stimuli angewendet.

Aus der WO 98/16086, die den nächstliegenden Stand der Technik darstelt, ist ein Kombinationssignal sowie eine Vorrichtung und ein Verfahren zur Erzeugung dieses Kombinationssignals bekannt.

Es ist daher die Aufgabe der vorliegenden Erfindung, ein verbessertes Konzept zum Betrieb eines Cochlea-Implantats zu schaffen, durch das ein Verhältnis zwischen einer Sprachverständlichkeit und einer Komplexität des Cochlea-Implantats gegenüber herkömmlichen Konzepten zum Betrieb von Cochlea-Implantaten verbessert werden kann.

Diese Aufgabe wird durch eine Vorrichtung zum Erzeugen eines Kombinationssignals basierend auf einem Audiosignal gemäß Anspruch 1, durch ein Kombinationssignal gemäß Anspruch 13, durch ein Verfahren gemäß Anspruch 19 sowie ein Computerprogrammprodukt gemäß Anspruch 20 gelöst.

Es wird ein Cochlea-Implantat zum Verarbeiten von Signalparametern, die für eine Ansteuerung des Cochlea-Implantats angepasst sind, die auf einem Audiosignal basieren bzw. ein Audiosignal repräsentieren, und die es ermöglichen, durch das Cochlea-Implantat eine Darstellung des Audiosignals zu erzeugen, offenbart. Ein Cochlea-Implantat umfasst eine Empfangsschnittstelle, die ausgelegt ist, um die Signalparameter zu empfangen, und eine Nervenstimulationseinrichtung zum Verarbeiten der Signalparameter, um basierend auf den Signalparametern Nervenzellen-Stimulationssignale zu erzeugen.

Die vorliegende Erfindung beruht auf der Erkenntnis, dass es vorteilhaft ist, wenn ein Cochlea-Implantat eine Empfangsschnittstelle aufweist, die ausgelegt ist, um Signalparameter zu empfangen, die ein durch das Cochlea-Implantat darzustellendes Audiosignal beschreiben. Gemäß dem Kerngedanken der vorliegenden Erfindung kann ein Cochlea-Implantat nämlich typischerweise Signalparameter, die ein Audiosignal beschreiben und die für eine Ansteuerung des Cochlea-Implantats angepasst sind, wesentlich effizienter verarbeiten als das Audiosignal selbst. Ein Cochlea-Implantat kann nämlich an die menschlichen Gehörnerven nicht das Audiosignal selbst weiterleiten, sondern regt die Hörnerven durch Nervenzellen-Stimulationssignale an, die aufgrund einer vergleichsweise einfachen Verarbeitung von den angepassten Signalparametern abgeleitet werden können.

Die erfindungsgemäße Verwendung von Signalparametern, die auf dem Audiosignal basieren, anstelle des Audiosignals selbst als Eingangssignal für ein Cochlea-Implantat ermöglicht wesentliche Verbesserungen bzw. Vereinfachungen bei einem Einsatz eines Cochlea-Implantats. Da die von dem Cochlea-Implantat empfangenen Signalparameter für ein Ansteuern des Cochlea-Implantats angepasst sind, muss das Cochlea-Implantat zu der Erzeugung der Nervenzellen-Stimulationssignale basieren auf den Signalparametern einen wesentlich geringeren Rechenaufwand aufwenden, als dies bei einer Erzeugung der Nervenzellen-Stimulationssignale aufgeräumt des Audiosignals selbst erforderlich wäre. Ein geringerer Rechenaufwand in dem Cochlea-Implantat resultiert wiederum in einem geringeren Stromverbrauch des Cochlea-Implantats, so dass bei gleichbleibender Kapazität einer Batterie eine wesentlich längere Betriebszeit des Cochlea-Implantats gewährleistet werden kann.

Andererseits kann die bei der Berechnung der Nervenzellen-Stimulationssignale eingesetzte Energie aber auch dazu verwendet werden, um bei gleichbleibender Betriebsdauer eine verbesserte Ansteuerung von Elektroden, die mit Nervenzellen des menschlichen Hörnervs gekoppelt sind, zu ermöglichen.

Ferner wird es durch einen Empfang von für eine Ansteuerung des Cochlea-Implantat angepassten Signalparametern durch das Cochlea-Implantat möglich, eine Berechnung der Signalparameter außerhalb des Cochlea-Implantats durchzuführen. Dadurch können die Signalparameter unter Verwendung einer komplexen Berechnungsvorschrift ermittelt werden, so dass eine gegenüber herkömmlichen. Cochlea-Implantaten verbesserte Sprachverständlichkeit erzielt werden kann.

Weiterhin sinkt der gesamte Hardwareaufwand im Vergleich zu herkömmlichen Systemen, da die Signalparameter für eine Mehrzahl von Patienten mit Cochlea-Implantaten zentral berechnet werden können. Jedes einzelne Cochlea-Implantat hingegen muss erfindungsgemäßer Weise lediglich eine Empfangsschnittstelle, die ausgelegt ist, um den angepassten Signalparameter zu empfangen, sowie eine Nervenstimulationseinrichtung zum Ableiten von Nervenzellen-Stimulationssignalen von den Signalparametern aufweisen. Eine komplexe Signalverarbeitungseinrichtung, die herkömmlicherweise in einem Cochlea-Implantat enthalten ist, um das Audiosignal an sich zu verarbeiten, kann hingegen entfallen. Somit können kostengünstige Cochlea-Implantate realisiert werden, die dennoch eine höchstmögliche Sprachverständlichkeit ermöglichen.

Im Übrigen wird darauf hingewiesen, dass eine Auslegung von Cochlea-Implantaten mit einer Empfangsschnittstelle für Signalparameter eine Normierung erlaubt, so dass Cochlea-Implantate von verschiedenen Herstellern mit den gleichen Gehör-angepassten Signalparametern arbeiten können.

Es wird ferner eine Vorrichtung zum Erzeugen eines Steuersignals für ein Cochlea-Implantat auf der Basis eines Audiosignals Offenbart. Eine Vorrichtung zum Erzeugen eines Steuersignals für ein Cochlea-Implantat umfasst einen Cochlea-Parameter-Extraktor zum Analysieren des Audiosignals, der ausgelegt ist, um basierend auf einer Analyse des Audiosignals, unter Verwendung eines Simulationsmodells für ein menschliches Gehör, Signalparameter als eine Eingabe-Information für das Cochlea-Implantat zu erzeugen. Eine Vorrichtung umfasst ferner eine Sende-Schnittstelle zum Senden der Signalparameter an das Cochlea-Implantat. Ferner ist die erfindungsgemäße Vorrichtung ausgebildet, um außerhalb eines Ohrs, in das das Cochlea-Implantat eingebracht ist, angeordnet zu sein.

Es ist der Kerngedanke der Vorrichtung zum Erzeugen eines Steuersignals für ein Cochlea-Implantat, Signalparameter als eine Eingabe-Information für das Cochlea-Implantat durch eine externe, nicht in den menschlichen Körper eingebrachte bzw. zum Betrieb außerhalb des menschlichen Körpers ausgelegte Vorrichtung zu berechnen. An der erfindungsgemäßen Vorrichtung wird daher eine Sende-Schnittstelle zum Senden der Signalparameter an das Cochlea-Implantat vorgesehen.

Die von der Einrichtung zum Erzeugen eines Steuersignals für ein Cochlea-Implantat extrahierten Signalparameter können für eine Ansteuerung von mehr als einem Cochlea-Implantat verwendet werden. Ferner ist es durch eine Körper-externe Anordnung der Vorrichtung zum Erzeugen eines Steuersignals für ein Cochlea-Implantat möglich, eine besonders hohe Rechenleistung bereitzustellen, so dass eine Eingabe-Information für das Cochlea-Implantat unter Verwendung eines Simulationsmodells für ein menschliches Gehör verarbeitet werden kann. Durch die Verwendung eines derartige Simulationsmodells, das beispielsweise bevorzugt eine mechanische Schwingungsanregung der Cochlea durch eine Bewegung der Basilarmembran beschreibt, kann eine Stimulation von Hörnerven durch das Cochlea-Implantat erreicht werden, die einen besonders realitätsnahen Höreindruck mit einer guten Sprachverständlichkeit erlaubt.

Die vorliegende Erfindung schafft eine Vorrichtung zum Erzeugen eines Kombinationssignals basierend auf einem Audiosignal, mit einer Einrichtung zum Bereitstellen von von dem Audiosignal abhängigen Signalparametern, die für eine Ansteuerung eines Cochlea-Implantats geeignet sind, bzw. die angepasst sind, um durch das Cochlea-Implantat eine Darstellung des Audiosignals zu erzeugen, und einem Signalkombinierer zum Kombinieren des Audiosignals und der Signalparameter, wodurch das Kombinationssignal erhalten wird.

Es hat sich nämlich gezeigt; dass es die Bildung eines Kombinationssignals, das sowohl das Audiosignal als auch Signalparameter, die für eine Ansteuerung eines Cochlea-Implantats angepasst sind, umfasst, ermöglicht, ein Audiosignal einer besonders großen Anzahl von Personen verfügbar zu machen. Das von der erfindungsgemäßen Vorrichtung gebildete Kombinationssignal kann nämlich mit geringem Aufwand sowohl für nicht-hörgeschädigte Personen als auch für Träger eines Cochlea-Implantats wiedergegeben werden. Die Erzeugung des Kombinationssignals mit Hilfe des Signalkombinierers ermöglicht es ferner einem Cochlea-Implantat, gegebenenfalls beide Signalanteile, also sowohl das Audiosignal an sich als auch die von dem Audiosignal abhängigen Signalparameter zu verwenden. In diesem Fall muss das Cochlea-Implantat beispielsweise aus dem Audiosignal an sich nur noch diejenigen Informationen zur Festlegung der Nervenzellen-Stimulationssignale berechnen, die durch die Signalparameter noch nicht mit ausreichender Präzision beschrieben sind. Dadurch ermöglicht die erfindungsgemäße Vorrichtung zum Erzeugen des Kombinationssignals, Cochlea-Implantate mit reduziertem Aufwand hinsichtlich der Signalverarbeitung zu entwickeln.

Ferner ermöglicht die erfindungsgemäße Vorrichtung zum Erzeugen des Kombinationssignals eine Bereitstellung von Signalen sowohl für herkömmliche Cochlea-Implantate, die das Audiosignal an sich verarbeiten, als auch für Cochlea-Implantate, deren Empfangsschnittstelle ausgelegt ist, um die Signalparameter zu empfangen. Hierbei kann das Cochlea-Implantat beispielsweise ausgelegt sein, um aus dem Kombinationssignal die Signalparameter zu extrahieren. Die Erzeugung eines Kombinationssignals ermöglicht somit eine bestmögliche Kompatibilität des erfindungsgemäßen Konzepts mit herkömmlichen Einrichtungen.

Ferner sei darauf hingewiesen, dass sich das Kombinationssignal für eine .besonders effiziente Übertragung, beispielsweise durch einen Rundfunksender, eignet.

Die vorliegende Erfindung schafft ferner ein Kombinationssignal mit einem Audiosignal und Signalparametern, die für eine Ansteuerung eines Cochlea-Implantats geeignet sind, bzw. die angepasst sind, um durch das Cochlea-Implantat eine Darstellung des Audiosignals zu erzeugen.

Die vorliegende Erfindung schafft ferner eine entsprechende Verfahren und ein Computerprogrammproduct in Analogie zu den oben beschriebenen Vorrichtungen. Das Verfahren und das Computerprogrammproduct bringen die gleichen Vorteile wie die beschriebenen Vorrichtungen.

Bevorzugte Ausführungsbeispiele der vorliegenden Erfindung werden nachfolgend Bezug nehmend auf die beiliegenden Figuren näher erläutert. Es zeigen:
- Fig. 1a: ein Blockschaltbild einer erfindungsgemäßen Vorrichtung zum Erzeugen eines Kombinationssig- nals basierend auf einem Audiosignal, gemäß einem Ausführungsbeispiel der vorliegenden Er- findung;
- Fig. 1b: ein Blockschaltbild eines Empfängers zum Bereitstellen von Signalparametern basierend auf einem Kombinationssignal;
- Fig. 2: ein Blockschaltbild eines Coch- lea-Implantats;
- Fig. 3a: eine schematische Darstellung einer Übertragungskette zu einem Cochlea- Implantat bei einer Signalanalyse vor einer Rund- funkübertragung oder Speicherung;
- Fig. 3b: eine schematische Darstellung eines Ablaufs bei einer Simulation eines menschlichen Gehörs sowie der bei der Simulation auftretenden Zwischen- und Endergebnisse;
- Fig. 4: eine schematische Darstellung eines erfindungsge- mäßen Kombinationssignals gemäß einem Ausführungsbeispiel der vorliegenden Erfindung;
- Fig. 5: ein Blockschaltbild einer Einrichtung zum Erzeugen eines Steuersignals für ein Cochlea-Implantat;
- Fig. 6a: ein Flussdiagramm eines Verfah- rens zum Betriebe eines Cochlea-Implantats;
- Fig. 6b: ein Flussdiagramm eines erfindungsgemäßen Verfah- rens zum Erzeugen eines Kombinationssignals, ge- mäß einem Ausführungsbeispiel der vorlie- genden Erfindung;
- Fig. 6c: ein Flussdiagramm eines Verfah- rens zum Erzeugen eines Steuersignals für ein Cochlea-Implantat;
- Fig. 7a: eine schematische Darstellung eines menschlichen Ohrs;
- Fig. 7b: eine schematische Darstellung eines menschlichen Ohrs, in das Elektroden eines Cochlea-Implantats eingebracht sind; und
- Fig. 8: eine schematische Darstellung von herkömmlichen Vorrichtungen zur Koppelung von Audiosignalen in ein Cochlea-Implantat.

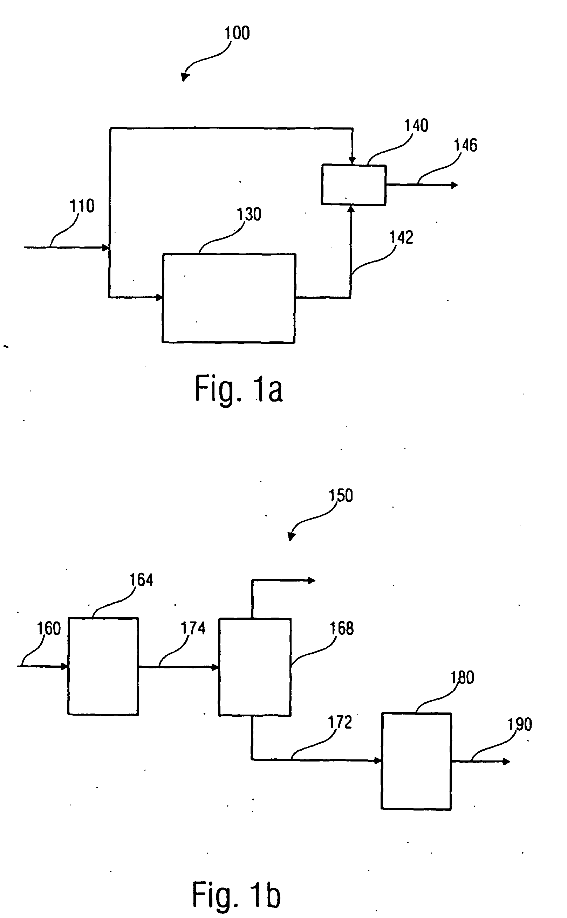

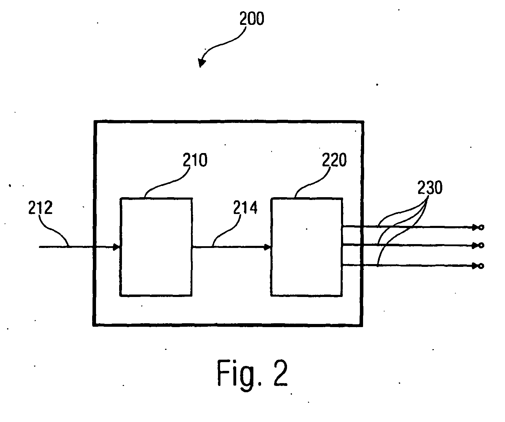

Im Folgenden werden Bezug nehmend auf die Fig. 1a, 1b und 2 Vorrichtungen beschrieben, die als Komponenten eingesetzt werden können, um das erfindungsgemäße Konzept, einem Cochlea-Implantat Signalparameter, die für eine Ansteuerung des Cochlea-Implantats angepasst sind, anstelle eines Audiosignals zuzuführen, zu realisieren.

Fig. 1a zeigt ein Blockschaltbild einer erfindungsgemäßen Vorrichtung zum Erzeugen eines Kombinationssignals, gemäß einem Ausführungsbeispiel der vorliegenden Erfindung. Die in Fig. 1a gezeigte Vorrichtung ist in ihrer Gesamtheit mit 100 bezeichnet. Die erfindungsgemäße Vorrichtung 100 ist ausgelegt, um ein Audiosignal 110 zu empfangen. Die erfindungsgemäße Vorrichtung 100 umfasst ferner eine Einrichtung 130 zum Bereitstellen von von dem Audiosignal abhängigen Signalparametern 142, die für eine Ansteuerung eines Cochlea-Implantats angepasst sind, und die es ermöglichen, durch das Cochlea-Implantat in einer wenig rechenaufwändigen Weise eine Darstellung eines Audiosignals zu erzeugen.

Die erfindungsgemäße Vorrichtung 100 umfasst ferner einen Signalkombinierer 140 zum Kombinieren des Audiosignals 110 und der von der Einrichtung 130 zum Bereitstellen von Signalparametern bereitgestellten Signalparameter 142. Der Signalkombinierer 140 liefert somit ein Kombinationssignal 146, in dem das Audiosignal 110 in die Signalparameter 142 kombiniert sind.

Fig. 1b zeigt ferner ein Blockschaltbild einer Einrichtung zum Bereitstellen von Signalparametern basierend auf einem Kombinationssignal. Die Vorrichtung der Fig. 1b ist in ihrer Gesamtheit mit 150 bezeichnet. Die Vorrichtung 150 ist ausgelegt, um ein Empfangssignal bzw. Kombinationssignal 160 zu empfangen, das zumindest ein Audiosignal und von dem Audiosignal abhängige Signalparameter, die für eine Ansteuerung eines Cochlea-Implantat angepasst sind, umfasst. Die Vorrichtung 150 umfasst optional eine Kanal-Auswahleinrichtung 164, die ausgelegt ist, um aus dem empfangenen Signal 160 einen Kanal auszuwählen, der ein Kombinationssignal bestehend aus einem Audiosignal und zugehörigen Signalparametern umfasst. In anderen Worten, die Kanal-Auswahleinrichtung 164 ist ausgelegt, um einen Kanal aus einer Mehrzahl von in dem empfangenen Signal 160 enthaltenen Kanälen an einen Signalparameter-Extraktor 168 weiterzuleiten. Der Signalparameter-Extraktor 168 ist ausgelegt, um aus dem ihm zugeführten Kombinationssignal 174 Signalparameter 172 zu extrahieren, die in dem empfangenen Signal 160 enthalten sind. Der Signalparameter-Extraktor 168 umfasst bevorzugt eine Datentrennungseinrichtung, die ausgelegt ist, um aus einem empfangenen Datenstrom 174 (der ein Kombinationssignal umfasst) einen Teildatenstrom 172 zu extrahieren. Die erfindungsgemäße Vorrichtung 150 umfasst ferner eine SignalparameterSendeeinrichtung 180, die ausgelegt ist, um die Signalparameter 172 zu empfangen und basierend darauf ein Signalparametersendesignal 190 zu erzeugen. Hierbei kann die Signalparametersendeeinrichtung eine Kanalcodierung und/oder eine Quellencodierung auf die Signalparameter 172 anwenden. Die Signalparametersendeeinrichtung kann ferner bevorzugt ausgelegt sein, um das Signalparametersendesignal 190 derart zu erzeugen, dass das Signalparametersendesignal 190 für eine drahtlose Übertragung geeignet ist. Das Signalparametersendesignal 190 kann beispielsweise derart erzeugt werden, dass es für eine Ansteuerung einer Induktionsschleife geeignet ist, mit deren Hilfe eine Koppelung zu einem Cochlea-Implantat hergestellt werden kann. Das Signalparametersendesignal 190 kann ferner aber auch für eine drahtlose Funkübertragung mit Hilfe einer elektromagnetischen Fernfeldkoppelung ausgelegt sein. Die Signalparametersendeeinrichtung 180 kann zu diesem Zweck eine Einrichtung zur Umsetzung auf eine Trägerfrequenz sowie eine Modulationseinrichtung umfassen. Es wird allerdings darauf hingewiesen, dass das Signalparametersendesignal 190 auch für eine drahtgebundene Übertragung des Signalparameter 172 ausgelegt sein kann.

Fig. 2 zeigt ein Blockschaltbild eines Cochlea-Implantats. Das in Fig. 2 gezeigte Cochlea-Implantat ist in seiner Gesamtheit mit 200 bezeichnet. Das Cochlea-Implantat 200 umfasst eine Empfangsschnittstelle 210, die ausgelegt ist, um Signalparameter zu empfangen, die für eine Ansteuerung des Cochlea-Implantats angepasst sind, die auf einem Audiosignal basieren, und die es ermöglichen, durch das Cochlea-Implantat eine Darstellung des Audiosignals zu erzeugen. Die Empfangsschnittstelle 210 ist ausgelegt, um ein Signalparameterempfangssignal 212 zu empfangen und basierend darauf Signalparameter 214 ableiten. Die Empfangsschnittstelle 210 ist dabei für eine Koppelung mit einer Empfangs-Induktionsschleife oder einer Antenne ausgelegt, um das Signalparameter-Empfangssignal 212 drahtlos zu empfangen. Alternativ kann die Empfangsschnittstelle 210 jedoch auch einen Leitungsempfänger umfassen, um ein drahtgebunden übertragenes Signalparameterempfangssignal 212 zu empfangen. Die Empfangsschnittstelle 210 kann ferner optional eine Einrichtung zur Frequenzumsetzung, einen Demodulator, eine Kanaldecodierer und/oder einen Quellendecodierer umfassen, die ausgelegt sind, um die Signalparameter 214 von dem Signalparameter-empfangssignal 212 abzuleiten.

Ferner kann die Empfangsschnittstelle 210 eine Auswahleinrichtung umfassen, um nur einen Teil der in dem Signalparameterempfangssignal 212 enthaltenen Information als Signalparameter 214 weiterzuleiten. Die Auswahleinrichtung kann hierbei ausgelegt sein, um nur einen Kanal von mehreren in dem Signalparameterempfangssignal 212 enthaltenen Kanälen zu verarbeiten. Ferner kann die Auswahleinrichtung ausgelegt sein, um die Signalparameter von einem gegebenenfalls zusätzlich in dem Signalparameterempfangssignal 212 enthaltenen Audiosignal-Anteil zu trennen, um somit zu ermöglichen, dass allein die Signalparameter an dem Ausgang der Empfangsschnittstelle 210 bereitstehen.

Das Cochlea-Implantat 200 umfasst ferner eine Nervenstimulationseinrichtung 220, die ausgelegt ist, um die Signalparameter 214 zu empfangen und zu verarbeiten, und um basierend auf den Signalparametern 214 Nervenzellen-Stimulationssignale 230 bereitzustellen. Die Nervenstimulationseinrichtung ist so ausgelegt, dass die Nervenzellen-Stimulationssignale 230 angepasst sind, um Elektroden des Cochlea-Implantats 200 zur Stimulation von Hörnerven des menschlichen Trägers des Cochlea-Implantats 200 anzuregen. Die Nervenzellen-Stimulationssignale 230 werden von der Nervenstimulationseinrichtung 220 derart berechnet, dass ein menschlicher Träger des Cochlea-Implantats 200 aufgrund der Anregung seiner Hörnerven durch die Nervenzellen-Stimulationssignale 230 einen Höreindruck des Audiosignals, auf dem die Signalparameter 214 basieren, gewinnt.

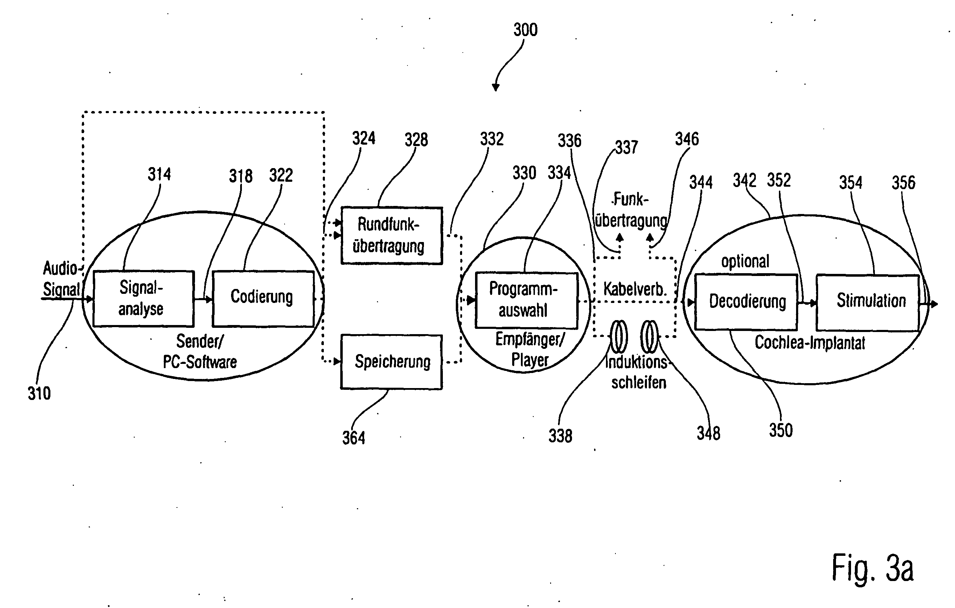

Fig. 3a zeigt eine schematische Darstellung einer Übertragungskette zu einem Cochlea-Implantat bei einer Signalanalyse vor einer Rundfunkübertragung oder Speicherung des Audiosignals. Die schematische Darstellung der Fig. 3a ist in ihrer Gesamtheit mit 300 bezeichnet.

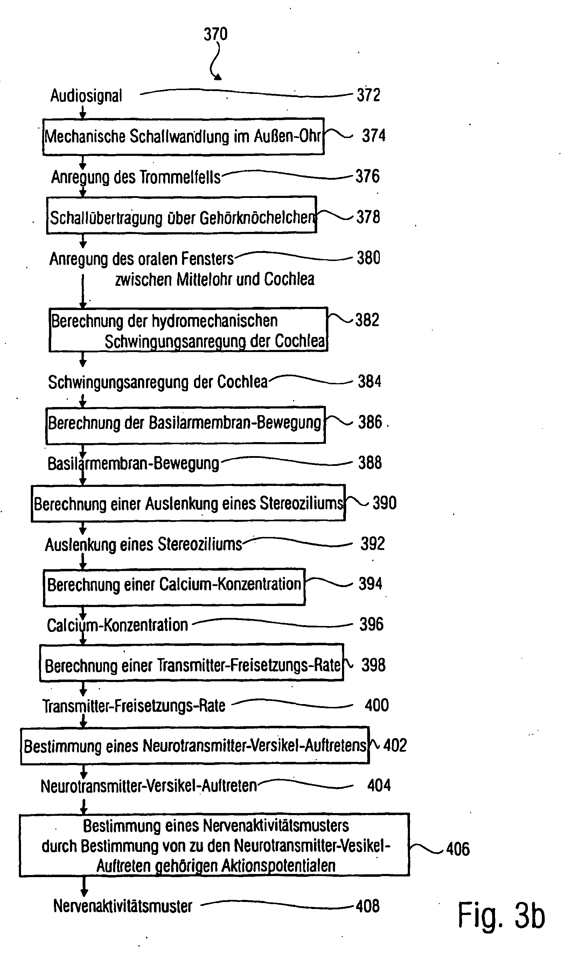

Die Übertragungskette 300 empfängt als Eingangsinformation ein Audiosignal 310. Es wird hierbei zunächst angenommen, dass eine Rundfunkübertragung des Audiosignals 310 erfolgt. Um eine möglichst effiziente Übertragung des Audiosignals zu ermöglichen und gleichzeitig eine besonders gute Sprachverständlichkeit für einen Träger eines Cochlea-Implantats sicherzustellen, wird das Audiosignal 310 bereits vor der Rundfunkübertragung einer Signalanalyse 314 zugeführt. Bei der Signalanalyse 314 könne Signalparameter 318 des Audiosignals 310 extrahiert werden, die für eine Ansteuerung eines Cochlea-Implantats angepasst werden. Die Signalanalyse 314 extrahiert bei dem gezeigten Ausführungsbeispiel Signalparameter 318, die zumindest eine innerhalb oder an einem Ausgang eines Simulationsmodells eines menschlichen Gehörs auftretende Größe beschreiben. In anderen Worten, im Rahmen der Signalanalyse 314 wird das Audiosignal 310 einem Simulationsmodell des menschlichen Gehörs als ein Eingangssignal zugeführt, und die Signalparameter 318 werden gewählt, um eine in dem Gehörmodell auftretende (relevante) physikalische Größe zu beschreiben. Weitere Details im Hinblick auf ein mögliches verwendetes Gehörmodell werden weiter unten mit Bezugnahme auf die Fig. 3b detailliert erläutert.

Die bei der Signalanalyse 314 entstehenden Signalparameter 318 werden dann einer optionalen Codierung 322 zugeführt. Die Codierung 322 kann beispielsweise eine Quellencodierung und/oder eine Kanalcodierung umfassen, wie diese aus der Nachrichtentechnik bekannt sind. Bei Verwendung der Codierung 322 entstehen somit codierte Signalparameter 324, die für eine Rundfunkübertragung 328 zur Verfügung stehen.

Gemäß dem Ansatz wird somit für eine Verbesserung der Sprachverständlichkeit bzw. der wahrgenommenen Klangqualität von Rundfunksendungen für Träger von Cochlea-Implantaten eine senderseitige Signalverarbeitung eingesetzt. Dabei wird die Tatsache ausgenutzt, dass bei einer Rundfunkübertragung senderseitig eine hohe Verarbeitungsleistung zur Verfügung gestellt werden kann. Durch die Signalanalyse 314 entstehen somit Signalparameter 318, die auch als vorverarbeitete Daten aufgefasst werden können. Die erhaltenen vorverarbeiteten Daten 318 können somit im Rahmen der Codierung 322 in geeigneter Weise codiert werden, um codierte Signalparameter 324 zu erhalten, und die codierten Signalparameter 324 können dann im Rahmen der Rundfunkübertragung 328 zusammen mit dem Rundfunksignal als eine Seiteninformation übertragen werden. Das im Rahmen der Rundfunkübertragung 328 übertragene Rundfunksignal umfasst dabei bevorzugt das Audiosignal 310 sowie die codierten Signalparameter 324.

Es sei angemerkt, dass speziell bei digital übertragenen Rundfunkprogrammen ein Hinzufügen einer Seiteninformation (bzw. von Seiteninformationen) zu den eigentlichen Ton- bzw. Bildsignalen auf einfache Weise möglich ist. Ferner sei darauf hingewiesen, dass im Rahmen der Rundfunkübertragung 328 somit ein Kombinationssignal bestehend aus dem Audiosignal 310 und den daraus extrahierten Signalparametern 318 übertragen werden kann, wobei die Erzeugung des Kombinationssignals 146 basierend auf dem Audiosignal 110 bereits mit Bezugnahme auf die Fig. 1a dargestellt wurde.

Die Rundfunkübertragung 328 kann im Übrigen ferner eine Frequenzumsetzung, eine Modulation und/oder eine Kanalcodierung zur Erhöhung einer Zuverlässigkeit der Übertragung umfassen.

Es sei ferner darauf hingewiesen, dass die codierten Signalparameter 324 mit dem (optional codierten) Audiosignal 310 auf verschiedene Weise kombiniert werden können. Im Rahmen einer digitalen Signalübertragung kann beispielsweise eine erste Gruppe von Bits innerhalb eines Bitrahmens dem codierten Audiosignal 310 zugeordnet sein, während den codierten Signalparametern 324 eine zweite Gruppe von Bits innerhalb des Bitrahmens zugeordnet ist. Eine Zuordnung kann hierbei statisch vorgegeben sein oder auch dynamisch je nach der anfallenden Dätenmenge des codierten Audiosignals und der codierten Signalparameter 324 erfolgen. Ferner ist es möglich, dem (gegebenenfalls codierten) Audiosignal 310 bei der Rundfunkübertragung 328 eine erste Gruppe von Unterträgern zuzuordnen, und ferner den codierten Signalparametern 324 eine zweite Gruppe von Unterträgern zuzuordnen, wobei die Unterträger der ersten Gruppe von Unterträgern und die Unterträger der zweiten Gruppe von Unterträgern zu demselben Hauptträger gehören. Bei einem weiteren Beispiel ist es möglich, das (gegebenenfalls codierte) Audiosignal 310 und die codierten Signalparameter 324 mit verschiedenen Trägerfrequenzen auszustrahlen.

Es wird hier allerdings darauf hingewiesen, dass zur Übertragung des (gegebenenfalls codierten) Audiosignals 310 und der codierten Signalparameter 324 auch jedes andere Übertragungsverfahren geeignet ist, das es erlaubt, empfängerseitig das codierte Audiosignal 310 und die codierten Signalparameter 324 wieder zu trennen.

Zusammenfassend lässt sich also festhalten, dass eine senderseitige Signalaufbereitung im Wesentlichen die folgenden Aufgaben erfüllt:
- Analyse 314 eines Audiosignals 310 bzw. Tonsignals unter Verwendung eines Software-Simulationsmodells für das menschliche Gehör,
- Extraktion von Parametern (auch als Signalparameter 318 bezeichnet), die für eine effiziente Ansteuerung eines Cochlea-Implantats geeignet sind, und
- Codierung 322 der Parameter 318 für eine effiziente Übertragung der Parameter 318 als eine Seiteninformation.

Im Falle einer Rundfunkübertragung umfasst die Übertragungskette 300 ferner einen Empfänger 330. Der Empfänger 330 empfängt ein im Rahmen der Rundfunkübertragung 328 übertragenes Rundfunkübertragungssignal 332 und ist ferner ausgelegt, um basierend auf dem Rundfunkübertragungssignal 332 mit Hilfe einer Programmauswahleinrichtung 334 eine zu empfangende übertragene Information auszuwählen. Die Programmauswahleinrichtung 334 kann dabei aus einer Mehrzahl von durch den Empfänger 330 empfangenen Rundfunkübertragungssignalen 332 einen vorgegebenen Kanal auswählen, in dem das zu empfangende (gegebenenfalls codierte) Audiosignal 310 zusammen mit den codierten Signalparametern 324 übertragen wird. Die Programmauswahleinrichtung 334 kann die entsprechende Auswahl durch eine Ausfilterung eines bestimmten Frequenzbereichs und/oder durch eine Auswahl von bestimmte Zeitschlitzen und/oder durch eine Auswahl von Signalanteilen in dem Rundfunkübertragungssignal 332, die mit einem bestimmten Code codiert sind, erreichen. Die Programmauswahleinrichtung 334 ist ausgelegt, um an ihrem Ausgang ein Signal 336 bereitzustellen, das die codierten Signalparameter 324 umfasst. Das Signal 336 an dem Ausgang der Programmauswahleinrichtung 334 kann ferner optional auch das codierte Audiosignal 310 (beispielsweise in Form eines Kombinationssignals) umfassen. Der Empfänger 330 umfasst ferner eine hier nicht gezeigte Signalsendeeinrichtung, die ausgelegt ist, um das Signal 336 für eine Übertragung zu einem Cochlea-Implantat 342 zu bearbeiten. Beispielsweise kann der Empfänger 330 ausgelegt sein, um eine Eunk-Übertragung des durch die Programmauswahleinrichtung 334 bereitgestellten Signals 336 zu dem Cochlea-Implantat 342 zu ermöglichen, indem der Empfänger 330 ein Sendesignal für eine Ansteuerung einer Antenne 337 bereitstellt. Ebenso gut kann der Empfänger 330 ausgelegt sein, um eine Induktionsschleife 338 zu speisen, die abgelegt ist, um eine Übertragung des Signals 336 zu dem Cochlea-Implantat 342 zu ermöglichen. Der Empfänger 330 kann außerdem alternativ einen Leitungstreiber umfassen, der eine Übertragung des Signals 336 mit den codierten Signalparametern 324 über eine kabelgebundene Verbindung zu dem Cochlea-Implantat 342 ermöglicht.

Das Cochlea-Implantat 342 ist bei dem gezeigten Ausführungsbeispiel ausgelegt, um ein Implantat-Empfangssignal 344 zu empfangen, das auf dem durch den Empfänger 330 an dem Ausgang der Programmauswahleinrichtung 334 bereitgestellten Signal 336 basiert. Das Cochlea-Implantat 342 kann zu diesem Zweck beispielsweise eine Empfangsantenne 346 aufweisen, um das Implantat-Empfangssignals 344, das die codierten Signalparameter 324 enthält, zu empfangen. Das Cochlea-Implantat 342 kann zusätzlich oder alternativ eine Implantat-Induktionsschleife 348 umfassen, um das Implantat-Empfangssignal 344 aufzunehmen. Ferner kann das Cochlea-Implantat optional einen Leitungsempfänger aufweisen, der ausgelegt ist, um das Signal 336 über eine Kabelverbindung von der Programmauswahleinrichtung 334 des Empfängers 330 als Implantat-Empfangssignal 344 zu empfangen.

Das Cochlea-Implantat 342 ist ausgelegt, um das Implantat-Empfangssignal 344 in einem Decoder 350 zu decodieren, um decodierte Signalparameter 352 zu erhalten. Das Cochlea-Implantat 342 ist derart ausgelegt, dass die decodierten Signalparameter 352 den bei der senderseitigen Signalanalyse 314 entstehenden Signalparametern 318 entsprechen. Das Cochlea-Implantat 342 bzw. der zugehörige Decoder 350 können allerdings optional so ausgelegt sein, dass als decodierte Signalparameter 352 nur eine echte Teilmenge der senderseitig bereitgestellten Signalparameter 318 zur Verfügung gestellt werden.

Ferner ist das Cochlea-Implantat 342 ausgelegt, um basierend auf den decodierten Signalparametern 352 Nervenzellen-Stimulationssignale bzw. Nervenzellen-Stimulationsimpulse 356 zu erzeugen, die ausgelegt sind, um über geeignete Elektroden Nervenzellen eines menschlichen (oder tierischen) Hörnervs anzuregen.

Zusammenfassend lässt sich somit festhalten, dass eine Signalverarbeitungseinrichtung eines Cochlea-Implantats 342 nur noch folgende zwei Schritte auszuführen hat:
- Decodierung 350 der (bei der Rundfunkübertragung 328 dem Rundfunkübertragungssignal 332 hinzugefügten) Seiteninformation, und
- Umwandlung 354 der empfangenen Parameter 352 in Stimulationsimpulse 356.

Es wird darauf hingewiesen, dass die Funktionalität des Cochlea-Implantats 342 ganz allgemein auch der Funktionalität des anhand von Fig. 2 beschriebenen Cochlea-Implantats 200 entsprechen kann. Der Decoder 350 ist hierbei als optional anzusehen, falls eine Decodierung der codierten Signalparameter 324 bereits innerhalb des Empfängers 330 erfolgt. In diesem Fall können somit uncodierte Signalparameter direkt drahtlos oder drahtgebunden zu dem Cochlea-Implantat 342 übertragen werden. Ferner wird darauf hingewiesen, dass das Cochlea-Implantat 342 ausgelegt sein kann, um ein im Rahmen der Rundfunkübertragung 328 auftretendes Seitensignal, das die codierten Signalparameter 324 enthält, von einem bei der Rundfunkübertragung 328 auftretenden Hauptsignal, das das codierte Audiosignal 310 enthält, zu trennen. In diesem Fall kann die entsprechende Funktionalität dem Empfänger 330 entfallen.

Im Übrigen kann ein Cochlea-Implantat 342 ferner optional auch eine Programmauswahleinrichtung aufweisen, deren Funktionalität der Funktionalität der Programmauswahleinrichtung 334 entspricht. Somit kann das Cochlea-Implantat 342 optional direkt das bei der Rundfunkübertragung 328 auftretende Rundfunkübertragungssignal 332 empfangen. Entscheidend ist hierbei lediglich, dass das Cochlea-Implantat 342 ausgelegt ist, um ein Signal zu empfangen, das (gegebenenfalls codierte) Signalparameter 324 enthält, die für eine Ansteuerung des Cochlea-Implantats angepasst sind, und die bevorzugt als Zwischengrößen oder Ausgangsgrößen eines Simulationsmodells eines menschlichen Gehörs auftreten.

Ferner wird darauf hingewiesen, dass an die Stelle der Rundfunkübertragung 328 eine Speicherung 364 der codierten Signalparameter 324 auf einem Speichermedium treten kann. Bei einem Speichermedium kann es sich beispielsweise um einen Halbleiterspeicher oder ein optisches oder magnetisches Speichermedium handeln, wie z. B. einen RAM-Speicher, einen ROM-Speicher, einen PROM-Speicher, einen EPROM-Speicher, einen EEPROM-Speicher, einen FLASH-Speicher, eine CDROM, eine DVD, ein Magnetband oder eine beliebige andere zur flüchtigen oder nichtflüchtigen Speicherung einer Information geeigneten Speichereinrichtung. An die Stelle des Empfängers 330 tritt hierbei ein Abspielgerät (Player), das ausgelegt ist, um die bei der Speicherung 364 in dem verwendeten Speichermedium abgelegte Information, die die codierten Signalparameter 324 umfasst, auszulesen und in der vorher beschriebenen Weise ein Signal 336, das die codierten Signalparameter 324 umfasst, an das Cochlea-Implantat 342 weiterzuleiten. Die Wiedergabeeinrichtung (Player) kann dabei ausgelegt sein, um eine vorgegebene Information aus dem bei der Speicherung 364 verwendeten Speichermedium auszuwählen.

Es wird hierbei darauf hingewiesen, dass im Rahmen der Speicherung 364 als Nutzinformation beispielsweise ausschließlich die codierten Signalparameter 324 gespeichert werden können. Andererseits ist es jedoch möglich, dass das Speichermedium neben den codierten Signalparametern auch das Audiosignal 310 in codierter Form umfasst. In diesem Fall kann das gleiche Speichermedium sowohl für eine Wiedergabe des Audiosignals an sich als auch für eine Wiedergabe der zu dem Audiosignal gehörigen Signalparameter 324 verwendet werden. Entweder die entsprechende Wiedergabeeinrichtung (Player) oder das Cochlea-Implantat 342 kann dabei ausgelegt sein, um aus den auf dem Speichermedium enthaltenen kombinierten Daten die codierten Signalparameter 324 zu extrahieren.

Somit kann festgehalten werden, dass ein Audiosignal 310 bzw. Tonsignal auf die gleiche Weise für eine Speicherung und/oder Wiedergabe in speziell für Träger von Cochlea-Implantaten angepassten mobilen Abspielgeräten aufbereitet werden kann, wie dies schon für eine Rundfunkübertragung beschrieben wurde. Anstelle einer Übertragung als Seiteninformation tritt hierbei die Speicherung 364 auf dem entsprechenden Medium, bei dem es sich beispielsweise um eine Speicherkarte oder eine Daten-CD handeln kann. Die Wiedergabeeinrichtung, auch als Abspielgerät oder Player bezeichnet, braucht nur die codierten Daten, die beispielsweise die codierten Signalparameter 324 beschreiben bzw. darstellen, an die Signalverarbeitungseinheit des Cochlea-Implantats zu liefern. Die Signalverarbeitungseinheit des Cochlea-Implantats 342 übernimmt dann, wie oben beschrieben, die weitere Verarbeitung, um Nervenzellen-Stimulationssignale 356 zu erzeugen.

Als Verbindung von dem Empfänger 330 bzw. von der Wiedergabeeinrichtung (Abspielgerät bzw. Player) zu dem Cochlea-Implantat 342 kann eine Kabelverbindung oder eine induktive Übertragung der digitalen Steuerdaten (also der codierten Signalparameter 324) dienen. Durch eine Erweiterung des Empfängers 330 bzw. der Wiedergabeeinrichtung um eine entsprechende Sendeeinheit und des Cochlea-Implantats 342 um eine entsprechende Empfangseinheit kann auch eine Funkstrecke für die Übertragung der codierten Signalparameter 324 zum Einsatz kommen.

Im Folgenden wird detailliert beschrieben, wie die Signalparameter 318 erzeugt werden können, da die Verwendung von geeigneten Signalparametern, die für eine Ansteuerung des Cochlea-Implantats 342 angepasst sind, einen wesentlichen Einfluss darauf hat, wie stark durch das erfindungsgemäße Konzept eine Sprachverständlichkeit verbessert werden kann. Es hat sich nämlich gezeigt, dass eine Verbesserung einer wahrgenommenen Klangqualität bei Patienten mit bereits eingesetzten Cochlea-Implantaten nur durch eine Verbesserung einer Signalverarbeitung bei einer Erzeugung der Nervenzellen-Stimulationssignale basierend auf dem zugrundeliegenden Audiosignal erzielt werden kann. Es hat sich hierbei gezeigt, dass ein Computersimulationsmodell die Reize der Nervenzellen eines gesunden Gehörs nachbilden kann, so dass Stärke und Abfolge der Simulationsimpulse bzw. Nervenzellen-Simulationssignale derart gewählt werden können, dass durch das Cochlea-Implantat möglichst ähnliche Reize hervorgerufen werden, wie dies anhand des Computersimulationsmodells für die Hörnerven eines isolierten Gehörs erwartet wird. Es hat sich allerdings ferner gezeigt, dass ein geeignetes Simulationsmodell einen derart hohen Rechenaufwand erfordert, dass zur Zeit eine Integration des Simulationsmodells in eine Signalverarbeitungseinrichtung eines Cochlea-Implantats nicht möglich ist.

In anderen Worten, eine wesentliche Verbesserungsmöglichkeit gegenüber herkömmlichen Cochlea-Implantaten besteht darin, ein Softwaresimulationsmodell für das menschliche Gehör im Rahmen der Erzeugung der Nervenzellen-Stimulationssignale 356 heranzuziehen. Hierbei wird es bevorzugt, dass sich das Softwaresimulationsmodell stark an der Physiologie des Ohres orientiert, wodurch das Softwaresimulationsmodell in der Lage ist, Schwingungsvorgänge, die ein Audiosignal 310 bzw. ein Schallsignal in einem gesunden Gehör bewirkt, mit großer Genauigkeit nachzubilden. Ein beispielhaftes geeignetes Softwaresimulationsmodell ist in der Dissertation "Ein psychophysiologisches Gehörmodell zur Nachbildung von Wahrnehmungsschwellen für die Audiocodierung" von F. Baumgarte (Dissertation, Universität Hannover, Deutschland; Februar 2000) beschrieben. Eine Erweiterung des genannten Softwaresimulationsmodells um ein Modell zur Umsetzung von Schwingungen in Nervensignale erlaubt es sogar, neuronale Aktivitäten eines Hörnervs zu simulieren. Für Einzelheiten diesbezüglich wird auf den Konferenzbeitrag "Using a physiological ear model for automatic melody transcription and sound source recognition" von T. Heinz und A. Brückmann (AES Convention, März 2003, Amsterdam) verwiesen.

Für eine Auswahl und Ansteuerung der Elektroden des Cochlea-Implantats 342 kann ein derartiges Simulationsmodell sehr hilfreich sein, da es eine Strategie bei der Auswahl und Ansteuerung der Elektroden ermöglicht, die neuronale Aktivitäten eines gesunden Gehörs durch eine elektrische Stimulation möglichst genau nachbildet. Allerdings erfordert ein derartiges Simulationsmodell, das im Übrigen in Software oder Hardware implementiert werden kann, einen derart hohen Rechenaufwand, dass eine Rechenleistung von gängigen Signalverarbeitungseinheiten (in Cochlea-Implantaten) hierfür nicht ausreicht.

Im Folgenden wird Bezug nehmend auf die Fig. 3b ein beispielhaftes Simulationsmodell näher beschrieben. Die Fig. 3b zeigt eine schematische Darstellung eines Ablaufs bei einer Simulation eines menschlichen Gehörs sowie der bei der Simulation auftretenden Zwischen- und Endergebnisse. Die graphische Darstellung der Fig. 3b ist in ihrer Gesamtheit mit 370 bezeichnet. Ein Audiosignal 372 dient als Eingangssignal für das Simulationsmodell 370. Basierend auf dem Audiosignal 372 kann in einem ersten Schritt 374 eine mechanische Schallwandung in einem Außenohr ausgewertet werden, wodurch eine Anregung 376 eines Trommelfels ermittelt werden kann. In einem zweiten Schritt 378 wird eine Schallübertragung über Gehörknöchelchen berechnet, wodurch aus der Anregung 376 des Trommelfells eine Anregung 380 eines ovalen Fensters zwischen einem Mittelohr und einer Cochlea berechnet werden kann. In einem dritten Schritt 382 wird eine hydromechanische Schwingungsanregung 384 der Cochlea berechnet. In einem vierten Schritt 386 kann aus der Schwingungsanregung 384 der Cochlea eine Basilarmembran-Bewegung 388 ermittelt werden. In einem fünften Schritt 390 wird von der Basilarmembranbewegung 388 auf eine Auslenkung 392 eines Stereoziliums gefolgert. Basierend auf der Auslenkung 392 des Stereoziliums kann sodann in einem sechsten Schritt 394 eine Kalziumkonzentration 396 in einer Haarzelle berechnet werden. Die Kalziumkonzentration 396 wird dann herangezogen, um in einem siebten Schritt 398 eine Transmitter-Freisetzungs-Rate 400 von Transmittersubstanzen zu berechnen. Basierend auf der Transmitter-Freisetzungs-Rate 400 wird in einem achten Schritt 402 ein Neurotransmitter-Vesikel auftreten 404 hergeleitet, das ein Auftreten von Neurotransmitter-Vesikeln beschreibt. Schließlich kann in einem neunten Schritt 406 ein Nervenaktivitätsmuster 408 von dem Neurotransmitter-Vesikel-Auftreten 404 abgeleitet werden. Das Nervenaktivitätsmuster 408 beschreibt hierbei näherungsweise eine bei einem gesunden Gehör auftretende Aktivität auf Nervenzellen eines (menschlichen oder tierischen) Hörnervs.

Das Nervenaktivitätsmuster 408 ist somit gut geeignet, um eine Aussage über eine bestmögliche Stimulation von Hörnerven durch ein Cochlea-Implantat 342 zu liefern.

Es wird hierbei darauf hingewiesen, dass im Rahmen des Simulationsmodells 370 mehrere der Schritte 374, 378, 382, 386, 390, 394, 398, 402, 406 zusammengefasst werden können, ohne ein entsprechendes Zwischenergebnis zu berechnen. In anderen Worten, es können mehrere Schritte in einem vereinfachten Schritt ohne Berechnung des in der graphischen Darstellung 370 gezeigten Zwischenschritts bearbeitet werden.

Es wird ferner darauf hingewiesen, dass als Signalparameter für eine Anwendung des erfindungsgemäßen Konzepts bevorzugt solche Zwischenergebnisse des in der schematische Darstellung 370 gezeigten Ablaufs verwendet werden, deren Berechnung aufgrund des Audiosignals mindestens 50 % des gesamten zur Berechnung der Nervenzellen-Stimulationssignale 356 aufgrund des Audiosignals nötigen Rechenaufwands erfordert. In anderen Worten, die Signalparameter sind bevorzugt so gewählt, dass die Berechnung der Nervenzellen-Stimulationssignale aufgrund der Signalparameter einen geringeren Rechenaufwand erfordert als eine Berechnung der Signalparameter aufgrund des Audiosignals.

Ferner hat es sich gezeigt, dass es vorteilhaft ist, wenn die als Signalparameter verwendeten Zwischengrößen bei der Berechnung der Nervenzellen-Stimulationssignale 356 aus dem Audiosignal 310 durch eine Vielzahl von verschiedenen Cochlea-Implantaten mit unterschiedlicher Anordnung der Elektroden und/oder unterschiedlichen Stimulationsmechanismen verwendet werden können.

Es hat sich diesbezüglich gezeigt, dass die Schwingungsanregung 384 der Cochlea, die die Anregung der in der Cochlea enthaltenen Flüssigkeit über das ovale Fenster zwischen Mittelohr und Cochlea beschreibt, in vorteilhafter Weise durch Signalparameter beschrieben werden kann. In anderen Worten, die Signalparameter 318 können beispielsweise gewählt sein, um die Schwingungsanregung der Cochlea zu beschreiben.

Ferner ist es in ähnlicher Weise vorteilhaft, wenn die Signalparameter 318 eine Bewegung bzw. Auslenkung 388 der Basilarmembran beschreiben. So können die Signalparameter 318 beispielsweise eine Stärke der Anregung der Basilarmembran für verschiedene Orte entlang der Basilarmembran beschreiben. Die Auslenkung kann hierbei direkt als Funktion einer Ortskoordinate oder in einer anderweitig linear oder nicht-linear transformierten Weise beschrieben werden. Die Signalparameter können somit die Anregung der Basilarmembran als Funktion von Ort und Zeit beschreiben.

Bei einem weiteren bevorzugten Ausführungsbeispiel beschreiben die Signalparameter 318 Auslenkungen 392 von mehreren Stereozilien, die an verschiedenen Positionen der Basilarmembran angeordnet sind. Wiederum könne die Signalparameter 318 die Auslenkung der Stereozilien direkt oder in einer transformierten Weise beschreiben.

In ähnlicher Weise ist es vorteilhaft, wenn die Signalparameter 318 die Kalziumkonzentration 396, die Transmitter-Freisetzungs-Rate 400 oder das Neurotransmitter-Vesikel-Auftreten 404 beschreiben.

Ferner wird es bei einem weiteren Ausführungsbeispiel bevorzugt, dass die Signalparameter 318 direkt das Nervenaktivitätsmuster 408, das die Anregung mehrerer Hörnerven charakterisiert, beschreiben. In diesem Fall geben die Signalparameter 318 direkt eine Aussage über einzelne Stimulationsimpulse, die das Cochlea-Implantat 342 für eine Anregung von Nervenzellen bereitzustellen hat.

Je nachdem, welche der genannten Zwischengrößen des Simulationsmodells 370 als Signalparameter 318 verwendet werden, ist es die Aufgabe des Cochlea-Implantats 342, aus der durch die Signalparameter 318 beschriebenen Zwischengröße des Gehörmodells 370 die für das Cochlea-Implantat passenden Nervenzellen-Stimulationssignale zu erzeugen. Es hat sich hierbei als besonders vorteilhaft erwiesen, als Signalparameter nicht direkt das Nervenaktivitätsmuster 408 bzw. Nervenzellen-Stimulationssignale zu übertragen, sondern bevorzugt Parameter, die eine Bewegung 388 der Basilarmembran oder eine Auslenkung 392 der Stereozilien beschreiben, zu übertragen, da in diesem Falle die Nervenzellen-Stimulationssignale für Cochlea-Implantate verschiedener Elektrodenanordnung von den Signalparametern in einer wenig rechenintensiven Weise abgeleitet werden können. Somit ermöglicht eine Beschreibung der Basilarmembranbewegung 388 oder einer Erregung 392 der Haarzellen eine zur Standardisierung geeignete Cochlea-Implantat-unabhängige Modellierung, wobei dennoch das einzelne Cochlea-Implantat 342 nur noch einen vergleichsweise geringen Anteil an dem gesamten Rechenaufwand für die Berechnung der Nervenzellen-Stimulationssignale durchführen muss.

Es sei ferner darauf hingewiesen, dass die Signalanalyse 314 weiterhin eine Vorverarbeitung des Audiosignals 310 umfassen kann. So kann das Audiosignal bzw. Tonsignal 310, sowohl in Verbindung mit einer Rundfunkübertragung 328 als auch in Verbindung mit einer Speicherung 364 der codierten Signalparameter 324, bereits vor der eigentlichen Anwendung des Simulationsmodells, das das menschliche Gehör beschreibt, für eine Ansteuerung des Cochlea-Implantats 342 optimiert werden kann.

Beispielsweise können bei der Bearbeitung eines Hörspiels im Rahmen der Signalanalyse 314 Hintergrundgeräusche oder Hintergrundmusik weggelassen werden, um eine Sprachverständlichkeit nicht unnötig zu beeinträchtigen. Zu diesem Zweck kann die Signalanalyseeinrichtung 314 beispielsweise Filter umfassen, die ein in dem Audiosignal 310 enthaltenes Sprachsignal betonen und Nicht-Sprachsignale dämpfen.

Ferner kann die Signalanalyseeinrichtung 314 eine Auswahleinrichtung zum Auswählen von einzelnen Kanälen des Audiosignals 310 umfassen, wenn das Audiosignal 310 in Form eines mehrkanaligen Signals vorliegt. In diesem Zusammenhang können aus dem gesamten Audiosignal 310, ähnlich wie bei einem Mischpult, nur diejenigen Audiosignalkanäle ausgewählt werden, die für einen Träger des Cochlea-Implantats 342 relevant sind. Somit ermöglicht es das erfindungsgemäße Konzept, im Rahmen einer Vorverarbeitung des Audiosignals 310 für einen Träger des Cochlea-Implantats 342 eine optimierte Information über das Audiosignal 310 in Form von Audiosignalparametern 342 bereitzustellen.

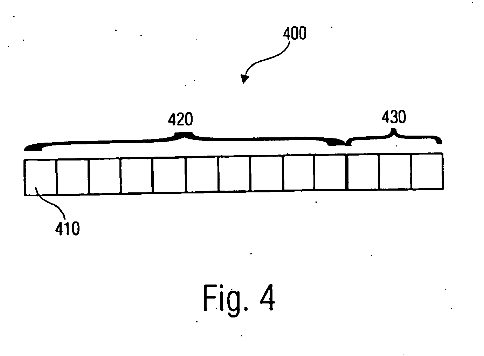

Fig. 4 zeigt eine schematische Darstellung eines erfindungsgemäßen Kombinationssignals gemäß einem Ausführungsbeispiel der vorliegenden Erfindung. Die graphische Darstellung der Fig. 4 ist in der Gesamtheit mit 400 bezeichnet. Ein erfindungsgemäßes Kombinationssignal ist hierbei symbolisch durch eine Mehrzahl von Bits 410 dargestellt. Ein erster Teil 420 des Kombinationssignals 400 beschreibt, beispielsweise in codierter oder uncodierter Form, ein Audiosignal zur Wiedergabe für einen Menschen mit einem gesunden Gehör. Ein zweiter Teil 430 des Kombinationssignals hingegen beschreibt in codierter oder uncodierter Form Signalparameter, die für eine Ansteuerung eines Cochlea-Implantats geeignet bzw. angepasst sind, um durch das Cochlea-Implantat eine Darstellung des durch den ersten Teil 420 des Kombinationssignals 400 beschriebenen Audiosignals zu erzeugen. Die in dem zweiten Teil 430 des Kombinationssignals 400 enthaltenen Signalparameter können beispielsweise eine Basilarmembran-Bewegung bzw. Basilarmembran-Auslenkung 388 beschreiben, die sich ergibt, wenn das Simulationsmodell 370 für das menschliche Gehör mit dem Audiosignal beaufschlagt wird. In ähnlicher Weise kann der zweite Teil 430 des Kombinationssignals eine Anregung von Haarzellen in einem Gehörmodell (beispielsweise in dem Gehörmodell 370) beschreiben. Ebenso kann der zweite Teil 430 des. Kombinationssignals 400 beispielsweise Stimulationsimpulse beschreiben, die bei einer entsprechenden Stimulation von Hörnerven eines Patienten eine Höreindruck ergeben, der dem zugrundeliegenden Audiosignal entspricht.

Es sie hierbei darauf hingewiesen, dass sowohl der erste Teil 420 als auch der zweite Teil 430 des erfindungsgemäßen Kombinationssignals in beliebiger Weise codiert sein können. Beispielsweise kann der erste Teil 420 des Kombinationssignals 400 und/oder der zweite Teil 430 des Kombinationssignals 400 eine Quellencodierung oder eine Kanalcodierung aufweisen. Im Übrigen ist es auch möglich, dass der erste Teil 420 und der zweite Teil 430 des Kombinationssignals 400 eine gemeinsame Quellencodierung oder eine gemeinsame Kanalcodierung aufweisen. Der erste Teil 420 des Kombinationssignals und der zweite Teil 430 des Kombinationssignals 400 können beispielsweise zeitlich koordiniert in verschiedenen Kanälen einer Übertragungseinrichtung übertragen werden. Die beiden Teile 420, 430 des Kombinationssignals 400 können ferner auch auf verschiedene Träger oder Unterträger desselben Nachrichtenkanals aufgeteilt sein. Ferner können der erste Teil 420 und der zweite Teil 430 des Kombinationssignals im Zeitmultiplex oder im Frequenzmultiplex zeitlich koordiniert übertragen werden. Einzelne Bits des ersten Teils 420 und des zweiten Teils 430 können ferner vermischt (interleaved) sein. Ein wesentliches Merkmal des erfindungsgemäßen Kombinationssignals 400 ist jedoch eine zeitlich koordinierte Übertragung des ersten Teils 420 und des zweiten Teils 430 des Kombinationssignals, wobei verschieden Modulationstypen und Bitaufteilungen verwendet werden können.

Im Übrigen wird darauf hingewiesen, dass das erfindungsgemasse Kombinationssignal 400 auch auf einem beliebigen Datenträger abgespeichert werden kann.

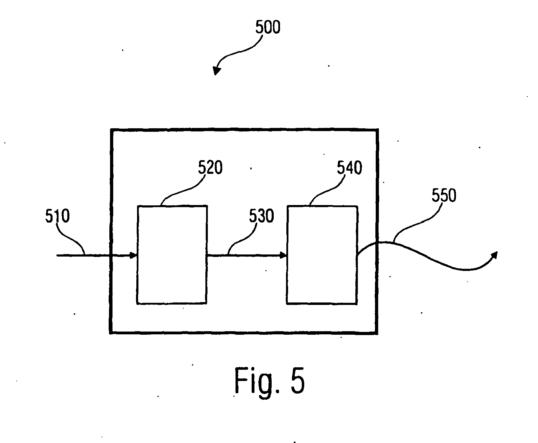

Fig. 5 zeigt ein Blockschaltbild einer Vorrichtung zum Erzeugen eines Steuersignals für ein Cochlea-Implantat. Das Blockschaltbild der Fig. 5 ist in seiner Gesamtheit mit 500 bezeichnet. Die Vorrichtung 500 ist ausgelegt, um ein Audiosignal 510 zu empfangen. Das Audiosignal 510 wird einem Cochlea-Parameter-Extraktor 520 zugeführt, der ausgelegt ist, um das Audiosignal zu analysieren und um basierend auf einer Analyse des Audiosignals Signalparameter 530 als eine Eingabeinformation für das Cochlea-Implantat zu erzeugen. Der Cochlea-Parameter-Extraktor 520 ist ausgelegt, um die Analyse des Audiosignals 510 unter Verwendung eines Simulationsmodells für ein menschliches Gehör durchzuführen. Die Signalparameter 530 sind hierbei Zwischenergebnisse oder Endergebnisse bei der Anwendung des Simulationsmodells. Als Simulationsmodell kann beispielsweise das in der Fig. 3b gezeigte Simulationsmodell 370 gewählt werden, wobei der Cochlea-Parameter-Extraktor 520 ausgelegt ist, um zumindest einen Teil des Simulationsmodells 370 abzuarbeiten bzw. um zumindest ein in dem Simulationsmodell 370 gezeigtes Zwischenergebnis 384, 388, 392, 396, 400, 404, 408 zu bestimmen und durch die Signalparameter 530 zu beschreiben.

In anderen Worten, die Signalparameter 530 beschreiben aufgrund der geeigneten Auslegung des Cochlea-Parameter-Extraktors beispielsweise eine Schwingungsanregung 384 der Cochlea, eine Basilarmembranbewegung 388, eine Auslenkung 392 eines Stereoziliums, eine Kalziumkonzentration 396, eine Transmitter-Freisetzungsrate 400, ein Neurotransmitter-Vesikel-Auftreten 404 oder ein Nervenaktivitätsmuster 408.

Die Vorrichtung 500 umfasst ferner eine Sendeschnittstelle 540 zum Senden der Signalparameter 530 an das Cochlea-Implantat. Die Sendeschnittstelle kann drahtlos oder drahtgebunden ausgelegt sein und beispielsweise eine Antenne, einen Leitungstreiber oder eine Sendeinduktionsschleife umfassen.

Es wird ferner darauf hingewiesen, dass die Vorrichtung 500 bevorzugt ausgebildet ist, um außerhalb eines Ohrs, in dem das Cochlea-Implantat eingebracht ist, angeordnet zu sein bzw. betrieben zu werden. Dies ist vorteilhaft, da die Vorrichtung 500 typischerweise eine hohe Rechenleistung aufweist und ausgelegt ist, um mehr als 50 % der zur Berechnung der Nervenzellen-Stimulationssignale aufgrund des Audiosignals nötigen Rechenleistung zu erbringen. Somit muss das Cochlea-Implantat nur eine geringere Rechenleistung aufweisen als die Vorrichtung 500. Durch diese Aufgabenteilung kann gewährleistet werden, dass das Cochlea-Implantat nur eine geringe Leistungsaufnahme aufweist und somit hinreichend klein für eine Implantation in den menschlichen Körper ausgelegt werden kann. Die Vorrichtung 500 ist daher, im Gegensatz zu dem Cochlea-Implantat, das bevorzugter Weise ausgelegt ist, um fest mit dem menschlichen Körper verbunden zu sein, bevorzugt trennbar von dem menschlichen Körper.

Während also das Cochlea-Implantat beispielsweise so gestaltet ist, dass es vollständig innerhalb einer Ohrmuschel oder eines Gehörgangs des menschlichen Ohres einschließlich des Mittelohrs und des Innenohrs eingebracht werden kann, ist die Vorrichtung 500 bevorzugt ausgelegt, um von dem menschlichen Körper ohne eine biologischen Eingriff trennbar zu sein.

Es wird im Übrigen bevorzugt, dass die Vorrichtung 500 als Telefon ausgebildet, bzw. Teil eines Telefons ist. In anderen Worten, die vorliegende Erfindung umfasst auch ein Telefon, das eine Vorrichtung 500 aufweist. In diesem Fall empfängt die erfindungsgemäße Vorrichtung 500 als Audiosignal 510 ein Telefon-Audiosignal, das beim herkömmlichen Telefon einem Lautsprecher bzw. einer Hörkapsel zugeführt würde. Ferner ist die Sendeschnittstelle 540 bei der genannten Anwendung bevorzugt ausgelegt, um eine Induktionsschleife anzusteuern, um die Signalparameter 530 zu einem Cochlea-Implantat zu übertragen. Es hat sich gezeigt, dass die Integration einer erfindungsgemäßen Vorrichtung 500 in ein Telefon wesentliche Vorteile mit sich bringt, da durch die erfindungsgemäße auf einem Simulationsmodell eines menschlichen Gehörs basierende Analyse des Audiosignals 510 die Sprachverständlichkeit beim Telefonieren ganz besonders stark erhöht werden kann. Da nämlich herkömmlicherweise ein Telefonsignal bereits von sich aus eine vergleichsweise niedrige Qualität aufweist, ist es für einen Patienten mit einem Cochlea-Implantat extrem schwierig, in Verbindung mit einem herkömmlichen Telefon und einem herkömmlichen Cochlea-Implantat eine telefonisch übermittelte Nachricht zu verstehen. Durch eine erfindungsgemäße Integration des Cochlea-Parameter-Extraktors 520 in ein Telefon kann im Gegensatz dazu die Übertragungsqualität des Telefons bei einer Bereitstellung der Signalparameter 530 mitberücksichtigt werden. Im Übrigen bietet eine erfindungsgemäße Kombination dem Benutzer bzw. dem Träger des Cochlea-Implantats wesentliche Vorteile hinsichtlich eines Benutzungskomforts.

Es wird ferner eine Mehrzahl von Verfahren, die das oben geschriebene Konzept einem Cochlea-Implantat Signalparameter zuzuführen, die für eine Ansteuerung des Cochlea-Implantats angepasst sind, offenbart.

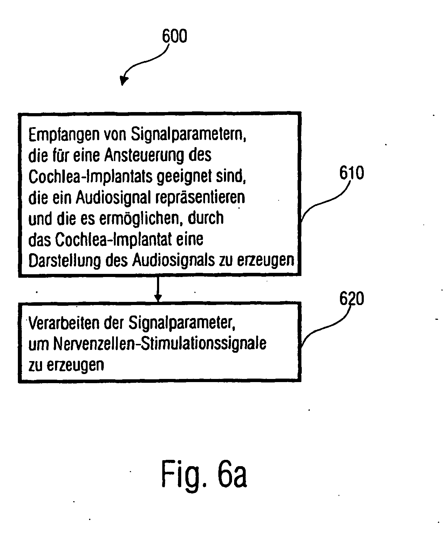

Fig. 6a zeigt ein Flussdiagramm eines Verfahrens zum Betreiben eines Cochlea-Implantats. Es wird hierbei davon ausgegangen, dass das Cochlea-Implantat in einer oben geschriebenen Weise ausgelegt ist, um Signalparameter zu verarbeiten, die für eine Ansteuerung des Cochlea-Implantats angepasst sind, die auf einem Audiosignal basieren und die es ermöglichen, durch das Cochlea-Implantat eine Darstellung des Audiosignals zu erzeugen. Das Verfahren ist in seiner Gesamtheit mit 600 bezeichnet. Das Verfahren 600 umfasst einen ersten Schritt 610 eines Empfangens von Signalparametern, die für eine Ansteuerung des Cochlea-Implantats geeignet sind, die ein Audiosignal repräsentieren, und die es ermöglichen, durch das Cochlea-Implantat eine Darstellung des Audiosignals zu erzeugen. Das erfindungsgemäße Verfahren 600 umfasst ferner einen zweiten Schritt 620 eines Verarbeitens der Signalparameter, um Nervenzellen-Stimulationssignale zu erzeugen.

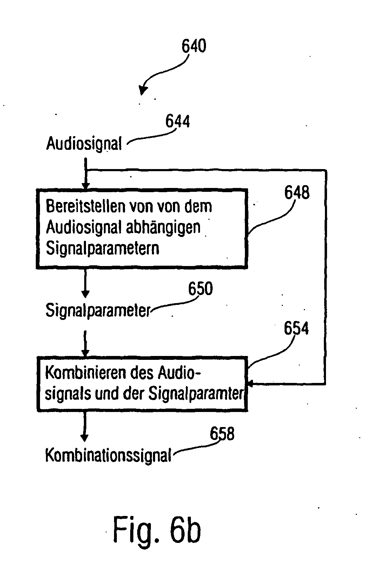

Fig. 6b zeigt ein Flussdiagramm eines erfindungsgemäßen Verfahrens zum Erzeugen eines Kombinationssignals, gemäß einem achten Ausführungsbeispiel der vorliegenden Erfindung. Das in der Fig. 6b gezeigte Verfahren ist in seiner Gesamtheit mit 640 bezeichnet. Das erfindungsgemäße Verfahren 640 empfängt als Eingangsgröße ein Audiosignal 644. Das Verfahren 640 umfasst einen ersten Schritt 648 eines Bereitstellens von von dem Audiosignal abhängigen Signalparametern 650, wie dies bereits oben ausführlich beschrieben wurde. Ein zweiter Schritt 654 umfasst ferner das Kombinieren des Audiosignals 644 und der zugehörigen Signalparameter 650, wodurch ein Kombinationssignal 658 erhalten wird.

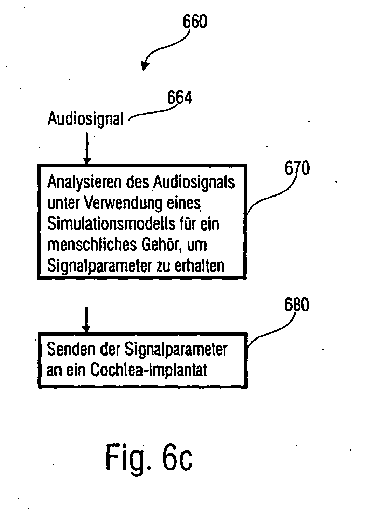

Die Fig. 6c zeigt ferner ein Flussdiagramm eines Verfahrens zum Erzeugen eines Steuersignals für ein Cochlea-Implantat. Das in der Fig. 6c gezeigte Verfahren ist in seiner Gesamtheit mit 660 bezeichnet. Das Verfahren 660 empfängt als Eingangssignal ein Audiosignal 664. Ein erster Schritt 670 umfasst ein Analysieren des Audiosignals unter Verwendung eines Simulationsmodells für ein menschliches Gehör, um zu dem Audiosignal 664 gehörige Signalparameter zu erhalten. Ein zweiter Schritt 680 umfasst ferner ein Senden der erhaltenen Signalparameter an ein Cochlea-Implantat.

Es sei hierbei darauf hingewiesen, dass die beschriebenen Verfahren 600, 640, 660 um all diejenigen Merkmale erweitert werden können, die bereits vorher mit Hinblick auf die Vorrichtungen beschrieben wurden.

Ferner kann das oben geschriebene Konzept durch ein Computerprogramm ausgeführt werden. Das konzept oder Verfahren kann, abhängig von den Gegebenheiten, in Hardware oder in Software implementiert werden. Die Implementation kann auf einem digitalen Speichermedium, beispielsweise einer Diskette, CD, DVD, oder einem Flash-Speichermedium, mit elektronisch auslesbaren Steuersignalen erfolgen, die so mit einem programmierbaren Computersystem zusammenwirken können, dass das entsprechende Verfahren ausgeführt wird. Allgemein besteht die Erfindung also auch in einem Computer-Programm-Produkt mit auf einem maschinenlesbaren Träger gespeicherten Programm-Code zur Durchführung des erfindungsgemäßen Verfahrens, wenn das Computer-Programm-Produkt auf einem Rechner abläuft. In anderen Worten ausgedrückt, kann die Erfindung somit als ein Computer-Programm-Produkt mit einem Programm-Code zur Durchführung des Verfahrens realisiert werden, wenn das Computer-Programm auf einem Computer abläuft.

Zusammenfassend lässt sich also festhalten, dass die vorliegende Erfindung die Nachteile von herkömmlichen Konzepten zur Erzeugung von Nervenzellen-Stimulationssignalen in einem Cochlea-Implantat überwindet Bei herkömmlichen Anwendungen, beispielsweise bei einem herkömmlichen Radio- oder Fernsehempfang oder bei einer Wiedergabe eines Tonsignals durch ein portables Medienwiedergabegerät muss das Audiosignal bzw. Tonsignal, welches eigentlich für Menschen mit einem gesunden Gehör aufbereitet ist, in der Signalverarbeitungseinheit des Cochlea-Implantats (in Nervensignale) umgewandelt werden. Lediglich beim Fernsehen können zusätzliche Untertitel zu einer Verbesserung des Gesamtverständnisses herangezogen werden.

Der Grundgedanke der vorliegenden Erfindung besteht also darin, dass der größte Teil der Signalverarbeitung bei einer Übertragung bzw. Speicherung von Audiosignalen bzw. Tonsignalen nicht in der Signalverarbeitungseinheit jedes einzelnen Cochlea-Implantats erfolgen muss, sondern mit leistungsfähigeren Prozessoren durchgeführt werden kann. Bei einer digitalen Rundfunkübertragung können die entsprechenden senderseitig verarbeiteten Signale (auch als Signalparameter bezeichnet) dann als Seiteninformation mitgesendet werden. Die Signalverarbeitungseinheiten (der Cochlea-Implantate) können dann mit geringem Rechenaufwand aus den Seiteninformationen die Stimulationsimpulse ableiten. Entsprechend können auch mobile Abspielgeräte entwickelt werden, die derart vorverarbeitete Signale speichern. Hierbei kann die Umwandlung bei der Erstellung entsprechender Tonträger, z. B. Hörbücher, oder am heimischen PC erfolgen, ähnlich wie bei der derzeit üblichen Codierung für MP3-Player.

Bei einer zentralen Signalaufbereitung in einem Rundfunksender oder bei einer Medienproduktion kann bevorzugter Weise beachtet werden, dass die erzeugte Seiteninformation möglichst universell verwendbar ist. Das heiß, die Seiteninformation sollte derart beschaffen sein, dass Cochlea-Implantate verschiedenen Typs ansteuerbar sind. Weiterhin sollte auch eine individuelle Optimierung der Weiterverarbeitung für einen einzelnen Träger eines Cochlea-Implantats möglich sein. Es sind jedoch auch Anwendungen möglich, bei denen eine individuelle Optimierung der Signalaufbereitung durchgeführt wird. Beispielsweise kann eine individuell konfigurierbare PC-Software für das Beschreiben von Medien für mobile Abspielgeräte ausgelegt werden.

Eine Abwandlung des oben beschriebenen Konzepts bzw. Verfahrens kann zur Verbesserung der Sprachqualität beim Telefonieren eingesetzt werden. Hier kann ein speziell für Träger von Cochlea-Implantaten entwickelter Telefonapparat die Aufbereitung des von einem Gesprächspartner kommenden Signals übernehmen. Sofern nämlich eine stationäre Anlage zum Einsatz kommt, kann diese die komplexen Signalverarbeitungsschritte übernehmen, so dass die Signalverarbeitungseinheit des Cochlea-Implantats wiederum, wie oben beschrieben, nur die Decodierung und Umwandlung auszuführen hat. Es zeigt sich also, dass das erfindungsgemäße Konzept vorteilhaft in einem Telefon eingesetzt werden kann.

Das vorliegende erfindungsgemäße Konzept ermöglicht somit eine deutliche Verbesserung der Sprachqualität bzw. der Sprachverständlichkeit bei Patienten mit einem Cochlea-Implantat. Ferner kann der innerhalb des Cochlea-Implantats benötigte Rechenaufwand deutlich verringert werden, so dass ein Cochlea-Implantat eine geringere Wärmeentwicklung und einen geringeren Stromverbrauch aufweist.

## Patentansprüche

1. Vorrichtung (100) zum Erzeugen eines Kombinationssignals (146) basierend auf einem Audiosignal (310), **dadurch gekennzeichnet, dass** die Vorrichtung folgende Merkmale umfasst:
eine Einrichtung (130) zum Bereitstellen von von dem Audiosignal abhängigen Signalparametern (142), die für eine Ansteuerung eines Cochlea-Implantats (200; 342) angepasst sind und ein durch das Cochlea-Implantat darzustellendes Audiosignal beschreiben; und
ein Signalkombinierer (140) zum Kombinieren des Audiosignals (110) und der Signalparameter (142), wodurch das Kombinationssignal (146) erhalten, wird;
wobei das Kombinationssignal sowohl das Audiosignal, das zur Wiedergabe für einen Menschen mit einem gesunden Gehör angepasst ist, als auch die Signalparameter, die für eine Ansteuerung eines Cochlea-Implantats angepasst sind und das durch das Cochlea-Implantat darzustellendes Audiosignal beschreiben, umfasst,
so dass das Kombinationssignal zur Wiedergabe sowohl für nicht-hörgeschädigte Personen als auch für Träger eines Cochlea-Implantats angepasst ist.

2. Vorrichtung (100) gemäß Anspruch 1, bei der die Einrichtung (130) zum Bereitstellen von von dem Audiosignal (110) abhängigen Signalparametern (142) einen Cochlea-Parameter-Extraktor umfasst, der ausgelegt ist, um basierend auf einer Analyse des Audiosignals (110) die Signalparameter als eine Eingabeinformation für das Cochlea-Implantat (200; 342) zu erzeugen.

3. Vorrichtung (100) gemäß Anspruch 2, bei der der Cochlea-Parameter-Extraktor ausgelegt ist, um die Signalparameter (142) durch Eingeben des Audiosignals (110) in ein Simulationsmodell (370) für ein menschliches Gehör zu berechnen, wobei das Simulationsmodell die Analyse des Audisignals beschreibt.

4. Vorrichtung (100) gemäß Anspruch 2 oder 3, wobei die Vorrichtung zum Erzeugen des Kombinationssignals (146) ausgelegt ist, um das Audiosignal (110) in Form von mehreren Audiosignal-Kanälen zu empfangen, die unterschiedliche Audiosignalinhalte aufweisen, und wobei die Vorrichtung ferner eine Kanalauswahleinrichtung umfasst, die ausgelegt ist, um für eine Analyse des Audiosignals mindestens einen Audiosignal-Kanal des Audiosignals auszuwählen, so dass die Analyse des Audiosignals nur auf eine Teilmenge der AudiosignalKanäle angewendet wird.

5. Vorrichtung (100) gemäß Anspruch 2 oder 3, wobei die Vorrichtung zum Erzeugen des Kombinationssignals (146) ausgelegt ist, um das Audiosignal (110) zu filtern, um Sprachsignale im Audiosignal gegenüber Hintergrundgeräuschen in dem Audiosignal zu betonen, um ein gefiltertes Audiosignal zu erhalten, und um das gefilterte Audiosignal für die Analyse zu verwenden.

6. Vorrichtung (100) gemäß einem der Ansprüche 1 bis 5, wobei die Vorrichtung zum Erzeugen des Kombinationssignals als ein Rundfunksender ausgebildet ist, der ausgelegt ist, um das Kombinationssignal (146) für eine Abstrahlung über eine Antenne aufzubereiten.

7. Vorrichtung (100) gemäß einem der Ansprüche 1 bis 6, bei der die Signalparameter (142) eine Bewegung (388) einer Basilarmembran eines menschlichen Ohrs aufgrund des Audiosignals (110) beschreiben.

8. Vorrichtung (100) gemäß einem der Ansprüche 1 bis 7, bei dem die Signalparameter (142) eine Anregung (392) von Haarzellen in einem menschlichen Ohr aufgrund des Audiosignals (110) beschreiben.

9. Vorrichtung (100) gemäß einem der Ansprüche 1 bis 8, bei dem die Signalparameter (142) Stimulationsimpulse (408) von Hörnerven eines menschlichen Ohrs aufgrund des Audiosignals beschreiben.

10. Vorrichtung gemäß einem der Ansprüche 1 bis 9, die ausgelegt ist, um das Kombinationssignal so zu erzeugen, dass ein erster Teil des Kombinationssignals das Audiosignal zur Wiedergabe für einen Menschen mit einem gesunden Gehör beschreibt, und so dass ein zweiter Teil des Kombinationssignals die Signalparameter beschreibt.

11. Vorrichtung gemäß einem der Ansprüche 1 bis 10, wobei die Vorrichtung ausgelegt ist, um die Signalparameter zu kodieren und um dem Audiosignal für eine Rundfunkübertragung eine erste Gruppe von Unterträgern zuzuordnen und den kodierten Signalparametern eine zweite Gruppe von Unterträgern zuzuordnen.

12. Vorrichtung gemäß Anspruch 11, wobei die Unterträger der ersten Gruppe von Unterträgern und die Unterträger der zweiten Gruppe von Unterträgern zu demselben Hauptträger gehören.

13. Kombinationssignal (400) bestehend aus einem Audiosignal (420) und von das Audiossignal abhängigen Signalparametern (430), **dadurch** gegenzeichnet, dass die Signalparameter für eine Ansteuerung eines Cochlea-Implantats (200; 342) angepasst sind und ein durch das Cochlea-Implantat darzustellendes Signal beschreiben, wobei das Kombinationssignal sowohl das Audiosignal, das zur Wiedergabe für einen Menschen mit einem gesunden Gehör ungepasst ist, als auch die Signalparameter, die für eine Ansteuerung eines Cochlea-Implantats angepasst sind und das durch das Cochlea-Implantat darzustellendes Signal beschreiben, umfasst,
so das das Kombinationssignal zur Wiedergabe sowohl für nicht-hörgeschädigte Personen als auch für Träger eines Cochlea-Implantats angepasst ist.

14. Kombinationssignal (400) gemäß Anspruch 13, wobei die Signalparameter (430) auf einer Analyse des Audiosignals (420) basieren und eine Eingabeinformation für das Cochlea-Implantat beschreiben.

15. Kombinationssignal (400) gemäß Anspruch 13 oder 14, wobei die Signalparameter (430) eine innerhalb eines Simulationsmodells (370) eines menschlichen Gehörs auftretende Größe oder eine Ausgangsgröße des Simulationsmodells beschreiben, die das Audiosignal repräsentieret, und die entsteht, wenn das Simulationsmodell (370) des menschlichen Gehörs durch das Audiosignal als Eingangssignal angeregt wird.

16. Kombinationssignal (400) gemäß einem der Ansprüche 13 bis 15, bei dem die Signalparameter (430) eine Bewegung (388) einer Basilarmembran eines menschlichen Ohrs aufgrund des Audiosignals (420) beschreiben.

17. Kombinationssignal (400) gemäß einem der Ansprüche 13 bis 16, bei dem die Signalparameter (430) eine Anregung (392) von Haarzellen in einem menschlichen Ohr aufgrund des Audiosignals (420) beschreiben.

18. Kombinationssignal (400) gemäß einem der Ansprüche 13 bis 17, bei dem die Signalparameter (430) Stimulationsimpulse (408) von Hörnerven eines menschlichen Ohrs aufgrund des Audiosignals (420) beschreiben.

19. Verfahren (640) zum Erzeugen eines Kombinationssignals basierend auf einem Audiosignal,
**dadurch gekennzeichnet, dass** das Verfahren folgende Schritte umfasst :
Bereitstellen (648) von von dem Audiosignal abhängigen Signalparametern, die für eine Ansteuerung eines Cochlea-Implantats angepasst sind, und ein durch das Cochlea-Implantat darzustellendes Signal beschreiben; und
Kombinieren (654) des Audiosignals und der Signalparameter, um das Kombinationssignal zu erhalten;
wobei das Kombinationssignal sowohl das Audiosignal, das zur Wiedergabe für einen Menschen mit einem gesunden Gehör angepasst ist, als auch die Signalparameter, die für eine Ansteuerung eines Cochlea-Implantats angepasst sind und das durch das Cochlea-Implantat darzustellendes Signal beschreiben, umfasst,
so das das Kombinationssignal zur Wiedergabe sowohl für nicht-hörgeschädigte Personen als auch für Träger eines Cochlea-Implantats angepasst ist.

20. Computerprogrammprodukt mit einem gespeicherten Programmcode, der zur Ausführung des Verfahrens gemäß Anspruch 19 angepasst ist.

## Claims

1. Apparatus (100) for producing a combination signal (146) based on an audio signal (310), **characterized in that** the apparatus comprises:
means (130) for providing signal parameters (142) which are dependent on the audio signal, are adapted to control a cochlea implant (200; 342), and describe an audio signal to be represented by the cochlea implant; and
a signal combiner (140) for combining the audio signal (110) and the signal parameters (142), the combination signal (146) being obtained in the process;
wherein the combination signal comprises both the audio signal adapted for reproduction for an individual with healthy hearing, and the signal parameters which are adapted to control a cochlea implant and describe the audio signal to be represented by the cochlea implant,
so that the combination signal for reproduction is adapted both for individuals whose hearing is not impaired and for carriers of a cochlea implant.

2. Apparatus (100) as claimed in claim 1, wherein the means (130) for providing signal parameters (142) dependent on the audio signal (110) comprises a cochlea parameter extractor configured to produce, on the basis of an analysis of the audio signal (110), the signal parameters as input information for the cochlea implant (200; 342).

3. Apparatus (100) as claimed in claim 2, wherein the cochlea parameter extractor is configured to calculate the signal parameters (142) by inputting the audio signal (110) to a simulation model (370) for human hearing, said simulation model describing the analysis of the audio signal.

4. Apparatus (100) as claimed in claim 2 or 3, the apparatus for producing the combination signal (146) being configured to receive the audio signal (110) in the form of several audio signal channels which comprise different audio signal contents, and the apparatus further comprising a channel selection means configured to select, for an analysis of the audio signal, at least one audio signal channel of the audio signal, so that the analysis of the audio signal is applied only to a subset of the audio signal channels.

5. Apparatus (100) as claimed in claim 2 or 3, the apparatus for producing the combination signal (146) being configured to filter the audio signal (110) so as to emphasize speech signals in the audio signal as against background noise in the audio signal, so as to obtain a filtered audio signal and to use the filtered audio signal for the analysis.

6. Apparatus (100) as claimed in any of claims 1 to 5, the apparatus for producing the combination signal being configured as a broadcast transmitter configured to condition the combination signal (146) for emission via an antenna.

7. Apparatus (100) as claimed in any of claims 1 to 6, wherein the signal parameters (142) describe a movement (388) of a basilar membrane of a human ear on the basis of the audio signal (110).

8. Apparatus (100) as claimed in any of claims 1 to 7, wherein the signal parameters (142) describe an excitation (392) of hair cells within a human ear on the basis of the audio signal (110).

9. Apparatus (100) as claimed in any of claims 1 to 8, wherein the signal parameters (142) describe stimulation pulses (408) of auditory nerves of a human ear on the basis of the audio signal.

10. Apparatus as claimed in any of claims 1 to 9, configured to produce the combination signal such that a first part of the combination signal describes the audio signal for reproduction for an individual with healthy hearing, and so that a second part of the combination signal describes the signal parameters.

11. Apparatus as claimed in any of claims 1 to 10, the apparatus being configured to encode the signal parameters and to associate a first group of subcarriers with the audio signal for broadcast transmission, and to associate a second group of subcarriers with the encoded signal parameters.

12. Apparatus as claimed in claim 11, wherein the subcarriers of the first group of subcarriers and the subcarriers of the second group of subcarriers belong to the same main carrier.

13. Combination signal (400) consisting of an audio signal (420) and of signal parameters (430) dependent on the audio signal, **characterized in that** the signal parameters are adapted to control a cochlea implant (200; 342) and describe a signal to be represented by the cochlea implant,
wherein the combination signal comprises both the audio signal adapted for reproduction for an individual with healthy hearing, and the signal parameters which are adapted to control a cochlea implant and describe the signal to be represented by the cochlea implant,
so that the combination signal for reproduction is adapted both for individuals whose hearing is not impaired and for carriers of a cochlea implant.

14. Combination signal (400) as claimed in claim 13, wherein the signal parameters (430) are based on an analysis of the audio signal (420) and describe input information for the cochlea implant.

15. Combination signal (400) as claimed in claim 13 or 14, wherein the signal parameters (430) describe a quantity occurring within a simulation model (370) of a human hearing system, or an output quantity of the simulation model, which represents the audio signal and arises when the simulation model (370) of the human hearing is excited by the audio signal as the input signal.

16. Combination signal (400) as claimed in any of claims 13 to 15, wherein the signal parameters (430) describe a movement (388) of a basilar membrane of a human ear on the basis of the audio signal (420).

17. Combination signal (400) as claimed in any of claims 13 to 16, wherein the signal parameters (430) describe an excitation (392) of hair cells within a human ear on the basis of the audio signal (420).

18. Combination signal (400) as claimed in any of claims 13 to 17, wherein the signal parameters (430) describe stimulation pulses (408) of auditory nerves of a human ear on the basis of the audio signal (420).

19. Method (640) for producing a combination signal based on an audio signal, **characterized in that** the method comprises:
providing (648) signal parameters which are dependent on the audio signal, are adapted to control a cochlea implant, and describe a signal to be represented by the cochlea implant; and
combining (654) the audio signal and the signal parameters so as to obtain the combination signal;
wherein the combination signal comprises both the audio signal adapted for reproduction for an individual with healthy hearing, and the signal parameters which are adapted to control a cochlea implant and describe the signal to be represented by the cochlea implant,
so that the combination signal for reproduction is adapted both for individuals whose hearing is not impaired and for carriers of a cochlea implant.

20. Computer program product comprising a stored program code adapted to perform the method as claimed in claim 19.

## Revendications

1. Dispositif (100) pour générer un signal composite (146) sur base d'un signal audio (310), **caractérisé par le fait que** le dispositif comporte les caractéristiques suivantes:
un moyen (130) destiné à mettre à disposition des paramètres de signal (142) dépendant du signal audio adaptés pour une activation d'un implant cochléaire (200; 342) et décrivant un signal audio à représenter par l'implant cochléaire; et
un combineur de signaux (140) destiné à combiner le signal audio (110) et le paramètre de signal (142), d'où est obtenu le signal composite (146);
le signal composite comprenant tant le signal audio adapté pour la reproduction pour un être humain avec une ouïe saine que les paramètres de signal adaptés pour une activation d'un implant cochléaire et décrivant le signal audio à représenter par l'implant cochléaire,
de sorte que le signal composite soit adapté pour la reproduction tant pour des personnes non handicapées de l'ouïe que pour des porteurs d'un implant cochléaire.

2. Dispositif (100) selon la revendication 1, dans lequel le moyen (130) destiné à mettre à disposition des paramètres de signal (142) dépendant du signal audio (110) comprend un extracteur de paramètres cochléaires qui est réalisé de manière à générer, sur base d'une analyse du signal audio (110), les paramètres de signal comme information d'entrée pour l'implant cochléaire (200; 342).

3. Dispositif (100) selon la revendication 2, dans lequel l'extracteur de paramètres cochléaires est réalisé de manière à calculer les paramètres de signal (142) par entrée du signal audio (110) dans un modèle de simulation (370) pour une ouïe humaine, le modèle de simulation décrivant l'analyse du signal audio.

4. Dispositif (100) selon la revendication 2 ou 3, dans lequel le dispositif destiné à générer le signal composite (146) est réalisé de manière à recevoir le signal audio (110) sous forme de plusieurs canaux de signal audio présentant différents contenus de signal audio, et le dispositif comprend par ailleurs un moyen de sélection de canal qui est réalisé de manière à sélectionner, pour une analyse du signal audio, au moins un canal du signal audio, de sorte que l'analyse du signal audio ne soit appliquée que sur une quantité partielle des canaux de signal audio.

5. Dispositif (100) selon la revendication 2 ou 3, dans lequel le dispositif destiné à générer le signal composite (146) est réalisé de manière à filtrer le signal audio (110), pour accentuer les signaux vocaux dans le signal audio par rapport au bruit de fond dans le signal audio, pour obtenir un signal audio filtré, et pour utiliser le signal audio filtré pour l'analyse.

6. Dispositif (100) selon l'une des revendications 1 à 5, dans lequel le dispositif destiné à générer le signal composite se présente sous forme d'émetteur radio qui est réalisé de manière à préparer le signal composite (146) pour une émission via une antenne.

7. Dispositif (100) selon l'une des revendications 1 à 6, dans lequel les paramètres de signal (142) décrivent un mouvement (388) d'une membrane basilaire d'une oreille humaine sur base du signal audio (110).

8. Dispositif (100) selon l'une des revendications 1 à 7, dans lequel les paramètres de signal (142) décrivent une activation (392) de cellules auditives dans une oreille humaine sur base du signal audio (110).

9. Dispositif (100) selon l'une des revendications 1 à 8, dans lequel les paramètres de signal (142) décrivent des impulsions de stimulation (408) de nerfs acoustiques d'une oreille humaine sur base du signal audio.

10. Dispositif selon l'une des revendications 1 à 9, réalisé de manière à générer le signal composite de sorte qu'une première partie du signal composite décrive le signal audio pour la reproduction pour un être humain avec une ouïe saine, et de sorte qu'une deuxième partie du signal composite décrive les paramètres de signal.

11. Dispositif selon l'une des revendications 1 à 10, dans lequel le dispositif est réalisé de manière à coder les paramètres de signal et à associer au signal audio pour une transmission par radio un premier groupe de sous-porteuses et à associer aux paramètres de signal codés un deuxième groupe de sous-porteuses.

12. Dispositif selon la revendication 11, dans lequel les sous-porteuses du premier groupe de sous-porteuses et les sous-porteuses du deuxième groupe de sous-porteuses appartiennent à la même porteuse principale.

13. Signal composite (400) composé d'un signal audio (420) et de paramètres de signal (430) dépendant du signal audio, **caractérisé par le fait que** les paramètres de signal sont adaptés pour une activation d'un implant cochléaire (200; 342) et décrivent un signal à représenter par l'implant cochléaire,
le signal composite comprenant tant le signal audio adapté pour la reproduction pour un être humain avec une ouïe saine que les paramètres de signal adaptés pour une activation d'un implant cochléaire et décrivant le signal à représenter par l'implant cochléaire,
de sorte que le signal composite soit adapté pour la reproduction tant pour des personnes non handicapées de l'ouïe que pour des porteurs d'un implant cochléaire.

14. Signal composite (400) selon la revendication 13, dans lequel les paramètres de signal (430) se basent sur une analyse du signal audio (420) et décrivent une information d'entrée pour l'implant cochléaire.

15. Signal composite (400) selon la revendication 13 ou 14, dans lequel les paramètres de signal (430) décrivent une grandeur se produisant dans un modèle de simulation (370) d'une ouïe humaine ou une grandeur de sortie du modèle de simulation représentant le signal audio, et qui se produit lorsque le modèle de simulation (370) de l'ouïe humaine est excité par le signal audio comme signal d'entrée.

16. Signal composite (400) selon l'une des revendications 13 à 15, dans lequel les paramètres de signal (430) décrivent un mouvement (388) d'une membrane basilaire d'une oreille humaine sur base du signal audio (420).

17. Signal composite (400) selon l'une des revendications 13 à 16, dans lequel les paramètres de signal (430) décrivent une activation (392) de cellules auditives dans une oreille humaine sur base du signal audio (420).

18. Signal composite (400) selon l'une des revendications 13 à 17, dans lequel les paramètres de signal (430) décrivent des impulsions de stimulation (408) de nerfs acoustiques d'une oreille humaine sur base du signal audio (420).

19. Procédé (640) pour générer un signal composite sur base d'un signal audio, **caractérisé par le fait que** le procédé comporte les étapes suivantes consistant à:
mettre à disposition (648) des paramètres de signal dépendant du signal audio adaptés pour une activation d'un implant cochléaire et décrivant un signal audio à représenter par l'implant cochléaire; et
combiner (654) le signal audio et le paramètre de signal, pour obtenir le signal composite;
le signal composite comprenant tant le signal audio adapté pour la reproduction pour un être humain avec une ouïe saine que les paramètres de signal adaptés pour une activation d'un implant cochléaire et décrivant le signal audio à représenter par l'implant cochléaire,
de sorte que le signal composite soit adapté pour la reproduction tant pour des personnes non handicapées de l'ouïe que pour des porteurs d'un implant cochléaire.

20. Programme d'ordinateur avec un code de programme mémorisé adapté pour la réalisation du procédé selon la revendication 19.
